# EUROPEAN PATENT APPLICATION

(11) **EP 2 074 994 A1**
(43) Date of publication of application: **01.07.2009**
(21) Application number: 09004643.4
(22) Date of filing: 18.07.2006
(51) Int. Cl.: A61K 31/365, A61K 31/436, A61K 31/192, A61K 31/56, A61K 31/60, A61K 31/7048, A61K 38/13, A61K 38/18, A61K 38/19, A61P 27/02, A61P 27/06, A61K 45/06

(54) **Enhanced ocular neuroprotection/neurostimulation**

(30) Priority: 18.07.2005 US 183355; 01.11.2005 US 263737
(62) Divisional of application: 06787603.7
(71) Applicant: Minu, L.L.C., Pittsboro, NC 27312 (US); Optivue L.L.P., Reading RG1 7SR (GB)
(72) Inventor: Peyman, Gholam A., Sun City AZ 85351 (US)
(74) Representative: Arends, William Gerrit

(57) **Abstract**

A macrolide-analogue with neuro-stimulatory and/or neuroprotective activity for use in partially or completely enhancing restoration of corneal sensation in the eye of a patient following laser assisted *in situ* keratomileusis (LASIK). The administration is non-systemic, localised ocular administration.

## Description

### Field of the Invention

The present invention extends generally to methods to enhance ocular neuro-protection and/or neuro-stimulation to reduce neurodegenerative changes that may be associated with ocular diseases, one example being glaucoma. The invention also extends to methods and compositions to enhance corneal sensation and/or reduce scarring after ocular surgery.

### Background Art

Several ocular and systemic diseases and treatments, including viral and bacterial infections, chemical burns and ocular surgery can cause corneal or ocular damage, including loss of corneal sensation, ocular scarring and/or nerve damage.

As such, methods and compositions that enhance a patient's condition prophylactically or therapeutically, or after ocular surgery are desirable.

### General

Those skilled in the art will appreciate that the invention described herein is susceptible to variations and modifications other than those specifically described. The invention includes all such variations and modifications. The invention also includes all of the steps, features, formulations and compounds referred to or indicated in the specification, individually or collectively and any and all combinations or any two or more of the steps or features.

Each document, reference, patent application or patent cited in this text is expressly incorporated herein in its entirety by reference, which means that it should be read and considered by the reader as part of this text. That the document, reference, patent application or patent cited in this text is not repeated in this text is merely for reasons of conciseness.

Any manufacturer's instructions, descriptions, product specifications, and product sheets for any products mentioned herein or in any document incorporated by reference herein, are hereby incorporated herein by reference, and may be employed in the practice of the invention.

The present invention is not to be limited in scope by any of the specific embodiments described herein. These embodiments are intended for the purpose of exemplification only.

The invention described herein may include one or more range of values (eg size, concentration etc). A range of values will be understood to include all values within the range, including the values defining the range, and values adjacent to the range which lead to the same or substantially the same outcome as the values immediately adjacent to that value which defines the boundary to the range.

Throughout this specification, unless the context requires otherwise, the word "comprise" or variations such as "comprises" or "comprising", will be understood to imply the inclusion of a stated integer or group of integers but not the exclusion of any other integer or group of integers. It is also noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

### Summary of the Invention

According to the present invention there is provided a method of enhancing ocular neuroprotection and/or neurostimulation in or to the eye of a patient, said method comprising the step of: locally administering in a non-systemic manner to the eye of a patient, a composition comprising at least one neuro-protective or neuro-stimulatory factor in a pharmaceutically effective formulation.

The invention further includes use of at least one neuro-protective or neuro-stimulatory factor for preparation of a medicament for enhancing ocular neuroprotection and/or neurostimulation in or to the eye of a patient.

In a further embodiment of the invention, there is also present in the pharmaceutically effective formulation at least one neurotrophin or neuropoietic factor.

The invention further includes use of at least one neuro-protective or neuro-stimulatory factor and at least one neurotrophin or neuropoietic factor for preparation of a medicament for enhancing ocular neuroprotection and/or neurostimulation in or to the eye of a patient.

In one embodiment the invention provides an ocular method for treating a post-ocular surgery patient or a trauma patient; comprising the step of: administering to said patient, a composition comprising at least one neuro-protective or neuro-stimulatory factor in a pharmaceutically effective formulation adapted for localized non-systemic ocular administration.

The invention further includes use of at least one neuro-protective or neuro-stimulatory factor for the preparation of a medicament for the treatment of a post-ocular surgery patient or a trauma patient.

Another embodiment is an ocular method comprising administering to a patient after ocular surgery a composition comprising at least one neuro-stimulatory or neuro-protective factor, which encompasses a macrolide or macrolide analog with neuro-stimulatory activity, in a pharmaceutically effective concentration and formulation for non-systemic localized ocular administration.

The invention further includes use of at least one neuro-stimulatory or neuro-protective factor, including a macrolide or macrolide analog with neuro-stimulatory or neuro-protective activity, for the preparation of a medicament for the treatment of a post-ocular surgery patient.

Another embodiment is an ocular method comprising administering to a patient prior to ocular surgery a composition comprising at least one neuro-stimulatory or neuro-protective factor, which encompasses a macrolide or macrolide analog with neuro-stimulatory or neuro-protective activity, in a pharmaceutically effective concentration and formulation for non-systemic localized ocular administration.

The invention further includes use of at least one neuro-stimulatory or neuro-protective factor, including a macrolide or macrolide analog with neuro-stimulatory or neuro-protective activity, for the preparation of a medicament for the treatment of a patient prior to ocular surgery.

Another embodiment is an ocular method comprising administering to a patient after ocular surgery a composition comprising at least one neuro-protective factor, which encompasses a macrolide or macrolide analog with neuro-protective activity, in a pharmaceutically effective concentration and formulation for non-systemic localized ocular administration.

According to the invention the composition may be ocularly administered such as through the topical route, subconjunctivally or intraocularly, administered by implantation in a device or a lens, or from a contact lens. The treatment may also be administered either alone or in conjunction with other therapy.

Further, the composition may be administered to the patient after corneal surgery such as laser-assisted in situ keratomileusis (LASIK), photorefractive keratectomy (PRK), laser assisted sub-epithelial keratomileusis (LASEK), total corneal transplant, or partial corneal transplant.

In a further embodiment, the invention provides a method for treating a post-ocular surgery patient with ocular scarring, comprising the step of: administering to said patient, a composition comprising at least one neuro-protective or neuro-stimulatory factor in a pharmaceutically effective formulation adapted for localized non-systemic ocular administration. According to this embodiment, the composition is administered to reduce or minimize scarring following any type of ocular surgery, including, but not limited to, glaucoma surgery, retinal detachment repair surgery, and corneal surgery.

The invention further includes use of at least one neuro-protective or neuro-stimulatory factor for the preparation of a medicament for the treatment of ocular scarring.

In a further embodiment, the invention provides a method for treating an ocular surgery patient at risk of ocular scarring, comprising the step of: administering to said patient a composition comprising at least one neuro-protective or neuro-stimulatory factor in a pharmaceutically effective formulation adapted for localized non-systemic ocular administration. In one embodiment of the invention, the composition is administered to a pre-ocular surgery patient at risk of developing ocular scarring, or to a post-ocular surgery patient exhibiting ocular scarring or at risk of developing ocular scarring. In one embodiment, at least one neuro-protective or neuro-stimulatory factor in a pharmaceutically effective formulation adapted for localized non-systemic ocular administration is prophylactically administered to an ocular surgery patient at risk of ocular scarring immediately prior to said patient undergoing ocular surgery. In one embodiment, at least one neuro-protective or neuro-stimulatory factor in a pharmaceutically effective formulation adapted for localized non-systemic ocular administration is prophylactically administered to a pre-ocular surgery patient at risk of ocular scarring less than 1 month, 2 weeks, 1 week, 3 days, 1 day, 18 hours, 15 hours, 12 hours, 9 hours, 6 hours, 3 hours, 1 hour, 30 minutes, 15 minutes, 10 minutes or 5 minutes prior to said patient undergoing ocular surgery. In one embodiment, at least one neuro-protective or neuro-stimulatory factor in a pharmaceutically effective formulation adapted for localized non-systemic ocular administration is administered to a post-ocular surgery patient at risk of developing ocular scarring or exhibiting ocular scarring for more than 2 years, 1 year, 9 months, 6 months, 3 months, 2 months, 1 month, 2 weeks, 1 week, 3 days, 1 day, 18 hours, 15 hours, 12 hours, 9 hours, 6 hours, 3 hours, 1 hour, 30 minutes, 15 minutes, 10 minutes or 5 minutes after said patient has undergone ocular surgery.

The invention further includes use of at least one neuro-protective or neuro-stimulatory factor for the preparation of a medicament for the prophylactic treatment of ocular scarring.

The invention further includes use of at least one neuro-protective or neuro-stimulatory factor for the preparation of a medicament for partially or completely restoring the loss of corneal sensation in the eye of a patient that occurs following a corneal procedure during which nerves are severed.

Another embodiment is an ocular method whereby a macrolide or macrolide analog is administered to a post-ocular surgery patient to reduce or minimize ocular scarring. The macrolide may be present as a component in a composition administered to provide a neuroprotective and/or neurostimulatory effect. Alternatively, the macrolide may be administered to reduce or minimize scarring following any type of ocular surgery, including but not limited to glaucoma surgery, retinal detachment repair surgery, and corneal surgery.

In yet another embodiment the invention provides a method for enhancing ocular nerve regeneration, and/or re-enervation in the eye of a patient, comprising the step of: administering to said patient, a composition comprising at least one neuro-protective or neuro-stimulatory factor in a pharmaceutically effective formulation adapted for localized non-systemic ocular administration.

The invention further includes use of at least one neuro-protective or neuro-stimulatory factor for the preparation of a medicament for enhancing ocular nerve regeneration, and/or re-enervation in the eye of a patient

The invention also provides a method for ameliorating or restoring the loss of corneal sensation in the eye of a patient that occurs following a corneal procedure during which nerves are severed, comprising the step of: administering to said patient, a composition comprising at least one neuro-protective or neuro-stimulatory factor in a pharmaceutically effective formulation adapted for localized non-systemic ocular administration.

Another embodiment is a method for administering a macrolide and/or a macrolide analog, with neuro protective and/or neurostimulatory activity, neurotrophin, and/or neuropoietic agent prophylactically to patients having or at risk for developing an ocular neurologic or neurosensory disease, or therapeutically to patients with an ocular neurologic or neurosensory disease, either alone or in conjunction with other therapy. Glaucoma is a non-limiting example of an ocular disease with a neuro-associated component. Retinitis pigmentosa is a non-limiting example of an ocular disease with a neurosensory component.

The invention further includes use of at least one macrolide and/or macrolide analog, with neuroprotective and/or nuerostimulatory activity, neurotrophin, and/or neuropoietic agent for preparation of a medicament for administration to a patient having or at risk for developing an ocular neurologic or neurosensory disease.

The invention further includes use of at least one neuro-protective or neuro-stimulatory factor for preparation of a medicament for administration prophylactically to a patient at risk for glaucoma or retinitis pigmentosa.

The invention also provides a method for ameliorating an ocular neurologic or neurosensory ailment, comprising the step of: administering to said patient, a composition comprising at least one neuro-protective or neuro-stimulatory factor in a pharmaceutically effective formulation adapted for localized non-systemic ocular administration.

The invention further includes use of at least one neuro-protective or neuro-stimulatory factor for preparation of a medicament for administration to a patient having an ocular neurologic or neurosensory ailment.

Alternatively, there is provided a method for prophylactically administering to a patient at risk of developing an ocular neurologic or neurosensory ailment, a composition comprising at least one neuro-protective or neuro-stimulatory factor in a pharmaceutically effective formulation adapted for localized non-systemic ocular administration.

The invention further includes use of at least one neuro-protective or neuro-stimulatory factor for preparation of a medicament for administration to a patient at risk of developing an ocular neurologic or neurosensory ailment.

The invention further provides a method of prophylactically administering to an individual with an ocular neurologic or neurosensory disease in a first eye but not in the second eye of said individual, a composition comprising at least one neuro-protective or neuro-stimulatory factor in a pharmaceutically effective formulation adapted for localized non-systemic ocular administration.

The invention further includes use of at least one neuro-protective or neuro-stimulatory factor for preparation of a medicament for administration to a second eye of a patient wherein the patient has an ocular neurologic or neurosensory disease in a first eye but not the second eye.

Another embodiment of the invention is a composition comprising at least one neuro-stimulatory factor in a pharmaceutically effective concentration and formulation for non-systemic localized ocular administration and effect. The composition contains macrolides with neuroprotective and/or neurostimulatory activity or may further be modified to contain one or more macrolides with neuroprotective and/or neurostimulatory activity, if not already present.

In another embodiment, the present invention provides compositions, comprising: at least one neuro-protective or neuro-stimulatory factor in a pharmaceutically effective concentration and formulation for localized non-systemic ocular administration and effect. The composition is suitable for use in one of the above methods. For example the concentration of the neuro-protective or neuro-stimulatory factor use in the composition will be effective for ameliorating the condition of a post-ocular surgery patient or a trauma patient or a patient suffering from an ocular neurologic or neurosensory ailment. Where the composition is used for a post ocular surgery patient or a trauma patient the composition is formulated for ameliorating or restoring the loss of corneal sensation in an eye. Compositions of the invention are desirably formulated for non-systemic administration.

Composition of the invention preferably contain at least a macrolide with neuroprotective and/or neurostimulatory activity, but may be modified to contain more than one macrolide, if not already present. They may be formulated with excipients for topical ocular administration, administration in an ocular device or delayed release matrix, administration by subconjunctival or intraocular injection, etc. It may be contained in an intraocular implant, an intraocular lens, or a contact lens.

Another embodiment is a method for administering at least one neuro-protective or neuro-stimulatory factor to patients having, or at risk for developing, an ocular neurologic or neurosensory disease, or to patients with an ocular neurologic or neurosensory disease, either alone or in conjunction with other therapy, wherein the disease is increased intraocular pressure, open angle glaucoma, and/or retinitis pigmentosa.

The invention further includes use of at least one neuro-protective or neuro-stimulatory factor for preparation of a medicament for ameliorating the condition of a patient with increased intraocular pressure, open angle glaucoma, and/or retinitis pigmentosa.

In a further embodiment of the invention, the neuro-protective or neuro-stimulatory factor comprises at least one neurotrophin selected from nerve growth factor-β (NGFβ), brain-derived neurotrophic factor (BDNF), neurotrophin 3 (NT-3), neurotrophin 4 (NT-4), neurotrophin 6, ciliary neurotrophic factor (CNTF), and/or glial cell derived neurotrophic factor (GDNF); and / or, at least one neuropoietic factor selected from leukemia inhibitory factor (LIF), ciliary neurotrophic factor (CNTF), oncostatin M, growth-promoting activity, or cardiotrophin 1.

The invention further includes use of at least one of nerve growth factor-β (NGFβ), brain-derived neurotrophic factor (BDNF), neurotrophin 3 (NT-3), neurotrophin 4 (NT-4), neurotrophin 6, ciliary neurotrophic factor (CNTF), glial cell derived neurotrophic factor (GDNF), leukemia inhibitory factor (LIF), ciliary neurotrophic factor (CNTF), oncostatin M, growth-promoting activity, and/or cardiotrophin 1 for preparation of a medicament for enhancing ocular neuroprotection and/or neurostimulation in or to the eye of a patient.

In a further embodiment, the invention comprises administering to a patient post-ocular surgery, pre-ocular surgery, with ocular scarring, or at risk of developing ocular scarring, a composition comprising at least one neuro-stimulatory or neuro-protective factor, in a pharmaceutically effective concentration and formulation for non-systemic localized ocular administration, wherein the neuro-stimulatory or neuro-protective factor is a macrolide and/or macrolide analog, with neurostimulatory and/or neuroprotective activity, and wherein the viability and/or activity of retinal sensory or ganglion cells is increased over viability and/or activity of the cells in the absence of the macrolide and/or macrolide analog.

The invention further includes use of at least one macrolide or macrolide analog with neuro-stimulatory or neuro-protective activity, for the preparation of a medicament for the administration to a post-ocular surgery patient and the increase of viability and/or activity of retinal sensory or ganglion cells.

In a further embodiment of.the invention, the neuro-protective or neuro-stimulatory factor is at least one of erythromycin, azithromycin, clarithromycin, lincomycin, dirithromycin, josamycin, spiramycin, diacetyl-midecamycin, troleandomycin, tylosin, roxithromycin, ABT-773, telithromycin, macrolides derived from leucomycins, lincosamides, or derivatives thereof.

The invention further includes use of at least one of erythromycin, azithromycin, clarithromycin, lincomycin, dirithromycin, josamycin, spiramycin, diacetyl-midecamycin, troleandomycin, tylosin, roxithromycin, ABT-773, telithromycin, macrolides derived from leucomycins, lincosamides, or derivatives thereof for preparation of a medicament for enhancing ocular neuroprotection and/or neurostimulation in or to the eye of a patient.

A further method of the invention comprises the steps of locally administering to an eye of an individual a biocompatible formulation of at least one neurostimulatory and/or neuroprotective macrolide or macrolide analog, optionally further including at least one neurotrophin or neuropoietic factor, under conditions to result in enhanced viability and/or activity of retinal sensory or ganglion cells over viability and/or activity of the cells in the absence of the macrolide or macrolide analog.

The invention further includes use of at least one neuro-stimulatory factor and a neurotrophin or neuropoietic factor, for the preparation of a medicament for the administration to a patient for the increased viability and/or activity of retinal sensory or ganglion cells.

Other aspects and advantages of the invention will become apparent to those skilled in the art from a review of the ensuing description.

### Detailed Disclosure of the Invention

As disclosed herein the applicant has determined that patient recovery after ocular surgery or other trauma can be enhancing by improving corneal sensation, ocular nerve regeneration, and/or re-enervation. According to this revelation, applicant provides methods that are capable of ameliorating the loss of corneal sensation that occurs following corneal procedures during which nerves are severed. The method also provides a means for reducing or minimising post-surgical scarring that could lead to corneal opacification, reduced vision, and/or other complications. In one embodiment of the invention, such a method includes use of a composition with a macrolide and/or macrolide analog, with neurostimulatory and/or neuroprotective activity, component. For example, it may be used to reduce or minimize scarring of the conjunctiva that occurs after glaucoma surgery or scarring that may lead to proliferative vitreal retinopathy (PVR) after retinal detachment repair surgery, or scarring that occurs after corneal surgery. While not being bound by a specific theory, a method to reduce or minimize post ocular-surgery scarring may enhance ocular sensation, nerve regeneration, and/or re-enervation, possibly by minimizing scar tissue that may impair nerve growth, nerve cell connections, etc. The method thus leads to enhanced recovery following ocular surgery.

According to the present invention there is provided a method of enhancing ocular neuroprotection and/or neurostimulation in or to the eye of a patient, said method comprising the step of locally administering in a non-systemic manner to the eye of a patient, a composition comprising at least one neuro-protective or neuro-stimulatory factor in a pharmaceutically effective formulation. The neuro-protective or neuro-stimulatory factor may be administered either alone or in combination with other neuro-stimulatory agents such as neurotrophins, neuropoietins, etc according to the methods described herein.

As used herein "a neuro-protective or neuro-stimulatory factor" is a composition that includes, at least, a macrolide and/or macrolide analog, with neurostimulatory and/or neuroprotective activity, neurotrophin, and/or neuropoietic agent. In one embodiment, the factor is present in a pharmaceutically effective concentration and it is formulated for localized non-systemic ocular administration. In one embodiment, the neuroprotective and/or neurostimulatory factor is, or includes, a macrolide such as cyclosporin A, tacrolimus, sirolimus, everolimus, pimecrolimus, zotarolimus, or a macrolide analog. Alternatively, they may have both activities. In some embodiments, one or more other agents may also be included in the composition, for example the composition may include one or more of the following: an antioxidant; a steroid; a non-steroidal anti-inflammatory drug; an antibiotic; an anti-proliferative agent; an anti-cell migration agent; a anti-prostaglandin; an anti-angiogenic agent; a vitamin; a mineral; a growth factor, or cytokine; or any other therapeutically ocular active agent.

Further, there is provided an ocular method comprising locally administering to an eye of an individual a pharmaceutically effective formulation of at least one neurostimulatory and/or neuroprotective macrolide or macrolide analog, under conditions to result in enhanced ocular neuroprotection and/or neurostimulation.

The phrase "pharmaceutically effective formulation" is used herein, and as known by a person skilled in the art, to refer to an amount or concentration of active agent either as an individual compound or in combination with other compounds that is sufficient to induce a prophylactic or ameliorative effect in a patient that is at risk of suffering from an ailment or is suffering from an ailment, respectively. This phrase should not be understood to mean that the dose must completely prevent or eradicate the ailment. What constitutes a pharmaceutically effective formulation will vary depending on, inter alia, the biopharmacological properties of the compound used in the methodology, the condition being treated, the frequency of administration, the mode of delivery, characteristics of the individual to be treated the severity of the ailment and the response of the patient. These are the types of factors that one skilled in the art will be aware of and will be able to account for when formulating compositions for a treatment as here in described.

In one embodiment, the invention provides an ocular method for administering to a post-ocular surgery patient or a trauma patient a composition comprising at least one neuro-protective and/or neuro-stimulatory factor in a pharmaceutically effective formulation adapted for localized non-systemic ocular administration.

Another embodiment is an ocular method comprising administering to a patient after ocular surgery a composition comprising at least one neuro-stimulatory and/or neuroprotective factor, which encompasses a macrolide and/or macrolide analog with neuro-stimulatory and/or neuroprotective activity, in a pharmaceutically effective concentration and formulation for non-systemic localized ocular administration.

According to the invention the composition may be ocularly administered such as through the topical route, subconjunctivally or intraocularly, administered by implantation in a device or a lens, such as from a contact lens, etc. The composition may also be administered either alone or in conjunction with other therapy.

In one aspect of the invention the patient will be suffering from corneal anesthesia. Corneal anesthesia is an unwanted consequence in some patients who have undergone an ocular surgical procedure. Such procedures include laser-assisted *in situ* keratomileusis (LASIK), photorefractive keratectomy (PRK), laser assisted sub-epithelial keratomileusis (LASEK), and corneal transplant (total or partial). In these types of procedures, the surgeon creates a micro-thin flap in the cornea and stroma to access the cornea. The stromal corneal flap may be created using a femtosecond computer-guided laser, or a hand-held microkeratome with an oscillating metal blade. The flap is then folded open to provide access to the cornea for the procedure, after which the flap is then returned to its original position where it seals without stitches. The flap promotes post-surgical healing, patient comfort, and improved vision. If the flap is not of the proper thickness (e.g., too thick, too thin, or irregular), the patient's healing and quality of vision may be compromised.

In creating the flap, the nerves that enervate the surface of the cornea are necessarily cut. One study reported that the number of sub-basal and stromal nerve fibre bundles in the corneal flap decreased 90% immediately following the surgery. Although the sub-basal nerve fibre bundles gradually returned, their number remained less than half of the pre-surgical number. The loss of corneal sensation caused by a decrease in the number of enervating nerves, and/or their function, may last up to about six months after the original procedure. Diabetic patients are particularly prone to decreased corneal nerve function, yet are a group of patients in frequent need of corneal transplants.

In a further form, the invention provides a method for administering to a post-ocular surgery patient with ocular scarring a composition comprising at least one neuro-protective and/or neuro-stimulatory factor in a pharmaceutically effective formulation adapted for localized non-systemic ocular administration.

According to this embodiment, the composition is administered to reduce or minimize scarring following any type of ocular surgery, including, but not limited to, glaucoma surgery, retinal detachment repair surgery, and corneal surgery. Glaucoma is a non-limiting example of an ocular disease with a neuro-associated component. Retinitis pigmentosa is a non-limiting example of an ocular disease with a neurosensory component.

Another embodiment is an ocular method whereby a macrolide and/or macrolide analog, with neurostimulatory and/or neuroprotective activity, is administered to a post-ocular surgery patient to reduce or minimize ocular scarring. The macrolide and/or analog may be present as a component in a composition administered to provide a neuroprotective and/or neurostimulatory effect. Alternatively, the macrolide may be administered to reduce or minimize scarring following any type of ocular surgery, including but not limited to glaucoma surgery, retinal detachment repair surgery, and corneal surgery.

After corneal surgery patients may experience problems relating to the loss of ocular sensitivity or sensation. For example, decreased ocular nerve function makes the cornea prone to trauma, which in turn can lead to infection. It reduces the usual blink mechanism that is required to keep the corneal surface moist, leading to drying and sloughing of the corneal epithelium. This, in turn, causes cloudiness of the flap, prones the flap to infection by enteral pathogens because of loss of barrier, and reduces vision.

In yet another embodiment the invention provides a method for ameliorating or restoring the loss of corneal sensation in the eye of a patient that occurs following a corneal procedure during which nerves are severed, comprising the step of: administering to said patient, a composition comprising at least one neuro-protective and/or neuro-stimulatory factor in a pharmaceutically effective formulation adapted for localized non-systemic ocular administration.

Alternatively the invention provides a method for enhancing ocular nerve regeneration, and/or re-enervation in the eye of a patient, comprising the step of: administering to said patient, a composition comprising at least one neuro-protective and/or neuro-stimulatory factor in a pharmaceutically effective formulation adapted for localized non-systemic ocular administration.

One embodiment of the invention locally administers one or more agents that enhance corneal sensation, possibly by nerve regeneration and/or enervation. In one embodiment, one or a combination of such macrolides, including macrolide analogues, is administered, the macrolide and/or analogue having neuro-stimulatory and/or neuro-protective activity. In another embodiment, one or a combination of macrolides is administered with one or more agent(s) that enhance corneal nerve stimulation and/or nerve protection. Such neuro-stimulatory agents may increase nerve cell quantity, functional quality, or combinations of these. One skilled in the art will appreciate that enhancement refers to any qualitative and/or quantitative improvement in corneal sensation and/or ocular neurological function following surgery regardless of degree.

### Methods for prophylactically or therapeutically treating a patient

In another form, the invention provides methods for ameliorating the condition of treating a patient with an ocular neurologic or neurosensory ailment or prophylactically administering to a patient at risk of getting ocular neurologic or neurosensory ailment.

Accordingly, another embodiment is a method for administering' a macrolide, and/or a macrolide analog, with neurostimulatory and/or neuroprotective activity, neurotrophin, and/or neuropoietic agent prophylactically to patients having or at risk for developing an ocular neurologic or neurosensory disease, or to patients with an ocular neurologic or neurosensory disease, either alone or in conjunction with other therapy. Glaucoma is a non-limiting example of an ocular disease with a neuro-associated component. Retinitis pigmentosa is a non-limiting example of an ocular disease with a neurosensory component.

Glaucoma is a general term for several types of a painless ocular condition that, left untreated, can result in partial or complete vision loss. It is characterized by elevated intraocular pressure, considered by one skilled in the art as a pressure greater than about 21.5 mm Hg. The higher the intraocular pressure, the greater the likelihood of optic nerve damage and visual field loss. In glaucoma monitoring or therapy, the neurodegenerative component, such as protection of retinal ganglion cells (RGC), should be considered in addition to therapy for increased intraocular pressure.

Known risk factors for glaucoma include age (elevated risk for individuals over age 60), race (elevated risk for African Americans over age 40), a family history of glaucoma, individuals with diabetes, severe nearsightedness, long-term corticosteroid use, previous eye injury, and/or increased intraocular pressure. One risk factor may suffice for prophylactic administration of a neuroprotective and/or neurostimulatory agent as described herein, and risk factors may alter over time, as known to one skilled in the art.

In monitoring, diagnosing, and/or treating patients with glaucoma, attainment of decreased intraocular pressure is a necessary but insufficient goal. This is because a component of glaucoma is neurologic damage to the optic nerve and ganglion cell death, so that its neurodegenerative aspects must be considered. Even patients with normal intraocular pressure may develop glaucoma-like changes. Further, retinal ganglion cells may be more sensitive to increased intraocular pressure, whereas other ocular cells may be better able to withstand increased intraocular pressure.

Retinitis pigmentosa is a general term that encompasses a disparate group of disorders of rods and cones. Because retinitis pigmentosa affects these retinal sensory structures, prophylactic or therapeutic administration of neuroprotective or neurostimulatory agents may reduce decreased visual field and other adverse effects.

In an alternate form the invention provides a method for ameliorating a patient with an ocular neurologic or neurosensory ailment, comprising the step of: administering to said patient, a composition comprising at least one neuro-protective and/or neuro-stimulatory factor in a pharmaceutically effective formulation adapted for localized non-systemic ocular administration.

In one embodiment, a composition containing a neuro-stimulatory or neuroprotective macrolide, macrolide analog, neurotrophin, and/or neuropoietic factor is administered to a patient having glaucoma, retinitis pigmentosa, or another neurosensory or neurodegenerative disease, either alone or in conjunction with other therapy.

Further, the invention provides a method for prophylactically administering to a patient at risk of developing an ocular neurologic or neurosensory ailment, a composition comprising at least one neuro-protective and/or neuro-stimulatory factor in a pharmaceutically effective formulation adapted for localized non-systemic ocular administration.

The invention also provides a method of prophylactically treating an individual with an ocular neurologic or neurosensory disease in a first eye but not in the second eye of said individual, comprising the steps of: administering to the second eye of said individual a composition comprising at least one neuro-protective and/or neuro-stimulatory factor in a pharmaceutically effective formulation adapted for localized non-systemic ocular administration.

In one embodiment, a composition containing a neuro-stimulatory and/or neuroprotective macrolide, macrolide analog, neurotrophin, and/or neuropoietic factor is prophylactically administered to patients at risk for developing glaucoma, retinitis pigmentosa, or other neurosensory or neurodegenerative disease, or may be administered to patients with glaucoma or retinitis pigmentosa, either alone or in conjunction with other therapy.

The inventive method may also be used to prevent or delay an increase in intraocular pressure, may reduce associated nerve loss, may confer protection on retinal sensory cells, and/or reduce apoptosis.

Ganglion cells in the retina (RGC) that have been damaged (e.g., by elevated intraocular pressure) undergo apoptosis, also referred to as programmed cell death. The macrolide tacrolimus, systemically administered, conferred neuroprotection on RGC by interfering with apoptotic mechanisms, as disclosed in Freeman and Grosskreutz (2000), Investigative Ophthalmology & Visual Science, 41: 1111, which is expressly incorporated by reference herein in its entirety. As a result of programmed cell death, RGC release compounds whose presence and/or concentration may result in toxicity, remove desirable agent and/or alter cell signaling; these compounds include cytokines, the excitatory neurotransmitter glutamate, Ca²+ binding proteins, FK 506 (tacrolimus) binding proteins, and others. Thus, ocular administration of a neurostimulatory and/or neuroprotective macrolide, macrolide analog, neurotrophin, and/or neuropoietic factor may reduce or inhibit subsequent effects of the released cytokines, glutamate, etc. that are part of the neurodegenerative processes associated with glaucoma and/or retinitis pigmentosa. For example, macrolides tacrolimus (FK 506) and cyclosporin A are potent immunosuppresants that inhibit T-cell activation by interfering with signal transduction. *In vitro,* tacrolimus binds to and inhibits the activity of the immunophilin FK 506-binding protein (FKBP), an isomerase that functions in signal transduction and cell communication. Reducing apoptotic mechanisms would reduce such processes, and thus protect or delay neurosensory impairment and/or neurodegenerative damage.

### Routes of Administration

Administration may be by any ocular route. In one embodiment, the composition is formulated for topical application. In another embodiment, the composition is formulated for intraocular application. In another embodiment, the composition is formulated for subconjunctival or intravitreal application. In another embodiment, the composition is in a delayed- or extended-release formulation. In another embodiment, the composition is formulated on or in a lens, including an intraocular lens, an implanted lens, an implantable lens or a contact lens. In another embodiment, the composition is formulated on or in an implanted or implantable ocular device. None of these formulations result in significant systemic absorption, so that there are no detrimental effects that may result with systemically administered macrolides and/or neurostimulatory factor(s).

One example is topical application, with the neurostimulatory and/or neuroprotective agent(s) administered in a formulation of eye drops, cream, ointment, gel, salve, etc.

Topical application of formulations of the invention may be as an *in situ* gellable aqueous formulation. Such a formulation comprises a gelling agent in a concentration effective to promote gelling upon contact with the eye or with lacrimal fluid in the exterior of the eye. Suitable gelling agents include, but are not limited to, thermosetting polymers such as tetra-substituted ethylene diamine block copolymers of ethylene oxide and propylene oxide (e.g., poloxamine); polycarbophil; and polysaccharides such as gellan, carrageenan (e.g., kappa-carrageenan and iota-carrageenan), chitosan and alginate gums.

The phrase "*in situ* gellable" as used herein embraces not only liquids of low viscosity that form gels upon contact with the eye or with lacrimal fluid in the exterior of the eye, but also more viscous liquids such as semi-fluid and thixotropic gels that exhibit substantially increased viscosity or gel stiffness upon administration to the eye. Indeed, it can be advantageous to formulate a formulation of the invention as a gel, to minimize loss of the formulation immediately upon administration, as a result, for example, of lacrimation caused by reflex blinking. Although it is preferred that such a formulation exhibit further increase in viscosity or gel stiffness upon administration, this is not absolutely required if the initial gel is sufficiently resistant to dissipation by lacrimal drainage to provide the effective residence time specified herein.

To prepare a topical formulation for the administration for ophthalmological disorders, a therapeutically effective amount of a composition of the invention is placed in an ophthalmological vehicle as is known in the art. The amount of the composition to be administered and the concentration of the composition in the topical formulations depend upon the diluent, delivery system or device selected, the clinical condition of the patient, the side effects and the stability of the compounds in the composition.

For topical administration, the concentration of composition administered may depend upon the particular patient, the underlying disease and its severity, the dosing frequency, and the active agent used etc., as known to one skilled in the art. Sample concentrations include, but are not limited to, about 0.5 mg/ml to about 2.5 mg/ml, about 1 mg/ml to about 5 mg/ml, about 5 mg/ml to about 10 mg/ml, about 10 mg/ml to about 15 mg/ml, about 15 mg/ml up to 30 mg/ml, etc.

Another example is intraocular injection with the neurostimulatory and/or neuroprotective agent administered subconjunctivally, intravitreally, retrobulbarly, within the crystalline lens via piercing the lens capsule as described in co-pending U.S. Patent application Serial No. 11/103,283 which is expressly incorporated by reference herein, etc.

According to this example, the composition may be intraocularly injected, for example, into the vitreous. When administering the composition by intravitreal injection, the active agents can be concentrated to minimise the volume for injection. For injection, a concentration less than about 20 mg/ml may be injected, and any amount may be effective depending upon the factors previously described. In an embodiment of this example, a dose of less than 7 mg/ml is administered, with doses of less than 6 mg/ml, 5 mg/ml, 4 mg/ml 3 mg/ml, 2 mg/ml and 1 mg/ml being administered in other embodiments. Sample concentrations include, but are not limited to, about 5 µg/ml to about 50 µg/ml; about 25 µg/ml to about 100 µg/ml; about 100 µg/ml to about 200 µg/ml; about 200 µg/ml to about 500 µg/ml; about 500 µg/ml to about 750 µg/ml; about 500 µg/ml up to 1 mg/ml; etc.

For example, in preparation for injection, topical alcaine may be applied to the ocular surface, followed by 5% povidone iodine. In such an example, a cotton-tipped applicator soaked in 4% lidocaine is then applied to the injection site, which is 4.0 mm posterior to the limbus in phakic eyes and 3.5 mm posterior to the limbus in pseudophakic eyes. A 27-gauge needle may be used for injection at the superior pars plana. Indirect ophthalmoscopy can be used to confirm proper intravitreal placement of the suspension.

In one embodiment of the invention, the syringe used in practicing this invention is suitably one which can accommodate a 21 to 30 gauge needle (eg a 23, 24, 25, 26 or 27 gauge needle) and the barrel may accommodate a small volume, for example 1.5 mL, or 0.5 mL. Although it is possible that the needle and syringe may be of the type where the needle is removable from the syringe, it is preferred that the arrangement is of a unitary syringe/needle construction. This would clearly limit the possibility of disengagement of the needle from the syringe. It is also preferred that the arrangement be tamper evident. The formulations of the present invention may therefore be provided in the form of a single unit dose in a pre-prepared syringe, ready for administration.

A suitable style of syringe is, for example, sold under the name of Uniject^{™} manufactured by Becton Dickinson and Company. In this style of syringe, the material is expelled through the needle into the eye by pressure applied to the sides of a pliable reservoir supplying the needle, rather than by a plunger. The construction of the reservoir and needle forms a single unit.

Another example provides the neurostimulatory and/or neuroprotective agent to the eye on or in a formulation such as a liposome, microsphere, microcapsule, biocompatible matrix, gel, polymer, nanoparticle, nanocapsule, etc.

Another example provides the neurostimulatory and/or neuroprotective agent on or in a device such as a device for transscleral delivery as described in co-pending U.S. patent application Serial No. 11/105,756, or another intraocular device using, for example, iontophoresis or another type of release mechanism (controlled or not controlled), as known by one skilled in the art.

Another example provides the neurostimulatory and/or neuroprotective agent in conjunction with gene therapy, as known by one skilled in the art.

### Compositions

Compositions described herein will have general application in the aforementioned methods but are not specifically limited to the methods, nor are the methods limited to the herein described compositions.

One embodiment of the invention is a composition comprising at least one neuro-stimulatory and/or neuro-protective factor in a pharmaceutically effective concentration and formulation for localized non-systemic ocular administration and effect.

In an alternate form the present invention also includes compositions, comprising: at least one neuro-protective and/or neuro-stimulatory factor in a pharmaceutically effective concentration and formulation for localized non-systemic ocular administration and effect. In one embodiment, composition is suitable for use in one of the above methods. For example the concentration of the neuro-protective and/or neuro-stimulatory factor use in the composition will be effective for ameliorating a post-ocular surgery patient or a trauma patient or a patient suffering from an ocular neurologic or neurosensory ailment. Where the composition is used in a post ocular surgery patient or a trauma patient the composition is formulated for ameliorating or restoring the loss of corneal sensation in an eye. Compositions of the invention are desirably formulated for non-systemic administration.

Composition of the invention preferably contains at least a macrolide, but may be modified to contain more than one macrolide, if not already present. They may be formulated with excipients for topical ocular administration, administration in an ocular device or delayed release matrix, administration by subconjunctival or intraocular injection, etc. It may be contained in an intraocular implant, an intraocular lens, or a contact lens.

The neurostimulatory and/or neuroprotective factor may be a macrolide, which may be cyclosporin A, tacrolimus, sirolimus, everolimus, pimecrolimus, zotarolimus or others; macrolide analog; neurotrophin; and/or a neuropoietic factor. Examples of macroldes or macrolide analogs include erythromycin, azithromycin, clarithromycin, lincomycin, dirithromycin, josamycin, spiramycin, diacetyl-midecamycin, troieandomycin, tylosin, roxithromycin, ABT-773, telithromycin, macrolides derived from leucomycins, lincosamides, or derivatives thereof.

Administration of such macrolides may be alone or may be in conjunction with other agents used to reduce intraocular pressure in patients with elevated intraocular pressure due to ocular hypertension or open-angle glaucoma. For example, administration may be included with a current drug regimen, or at different intervals than a current regimen, or for a set duration, etc; all these are examples of administration in conjunction with other agent. Examples of known drugs include, but are not limited to, Diamox® (acetazolamide (N-(5-sulfamoyl-1,3,4-thiadiazol-2-yl)acetamide), an inhibitor of carbonic anhydrase, Wyeth, Madison NJ); Timoptic® (timolol maleate ophthalmic solution, Merck, Whitehouse Station NJ), Xalatan® (latanaprost ophthalmic solution, Pfizer, Groton CA); Copaxone® (Teva Pharmaceuticals, Petah Tiqva, Israel); Memantine (Allergan, Irvine CA); Alphagan® P (brimonidine tartrate ophthalmic solution; Allergan); and others known to one skilled in the art. In this embodiment, the inventive method may use such macrolides to potentiate the action of current treatments. For example, acetazolamide may assist in normal polarization of cell membranes, so the effect of local ocular treament with acetazolamide and a neuroprotective macrolide or agent, is reduced apoptotic effects and normalized polarization of sensory retinal ganglion cells. The dual action may be additive or synergistic.

Macrolides encompassed by the invention are those known by one skilled in the art, as well as analogs and derivatives. These are disclosed in, for example, co-pending U.S. Patent Application Serial Nos. 10/667,161 and 10/752,124. Macrolides and their analogues that may be administered include the following.

Cyclosporin A (cyclosporine, topical formulation Arrestase®, Allergan Inc.) is a cyclic peptide produced by Trichoderma polysporum. It is available commercially, for example, from Sigma-Aldrich (St. Louis MO). It is an immunosuppressant and acts in a particular subset of T lymphocytes, the helper T cells. Cyclosporin A exerts an immunosuppressant effect by inhibiting production of the cytokine interleukin 2. Each of Cyclosporin A and tacrolimus, another immunosuppressant, produces significant renal and hepatic toxicity when each is administered systemically; because of this toxicity, they are not administered together. The use of Cyclosporin A as a specific medicament for treatment of ocular disease with reduced toxicity is described in co-pending U.S. Patent application Serial No. 10/289,772.

Tacrolimus (Prograf®, previously known as FK506), a macrolide immunosuppressant produced by Streptomyces tsukubaensis, is a tricyclo hydrophobic compound that is practically insoluble in water, but is freely soluble in ethanol and is very soluble in methanol and chloroform. It is available under prescription as either capsules for oral administration or as a sterile solution for intravenous administration. The solution contains the equivalent of 5 mg anhydrous tacrolimus in 1 ml of polyoxyl 60 hydrogenated castor oil (HCO-60), 200 mg, and dehydrated alcohol (USP, 80.0%v/v), and must be diluted with a solution of 0.9% NaCl or 5% dextrose before use.

Sirolimus, also known as rapamycin, RAPA, and Rapamune®, is a triene macrolide antibiotic derived from Streptomyces hydroscopicus and originally developed as an antifungal agent. Subsequently, it has shown anti-inflammatory, anti-tumor, and immunosuppressive properties. Pimecrolimus, also known as ascomycin, Immunomycin, and FR-900520, is an ethyl analog of tacrolimus and has strong immunosuppressant properties. It inhibits Th1 and Th2 cytokines, and preferentially inhibits activation of mast cells, and is used to treat contact dermatitis and other dermatological conditions. Sirolimus and pimecrolimus are commercially available, e.g., A.G. Scientific, Inc. (San Diego, CA).

Regarding its immunosuppressive potential, sirolimus has some synergetic effect with Cyclosporin A. It has been reported that sirolimus has a different mode of action compared to Cyclosporin A and tacrolimus. All three agents are immunosuppressants which affect the action of immune cell modulators (cytokines), but do not affect the immune cells themselves. However, while all three agents affect immune cell modulators, they do so differently: Cyclosporin A and tacrolimus prevent synthesis of cytokine messengers, specifically interleukin-2, while sirolimus acts on cytokine that has already been synthesized, preventing it from reaching immune cells.

Sirolimus inhibits inflammation by acting on both T-lymphocytes and dendritic cells. The latter are the first cells to recognize antigens. Sirolimus blocks the growth of dendritic cells and a number of other cells, such as tumors and endothelial cells, which are activated by the tumor cell releasing vascular endothelial growth factor (VEGF). VEGF is a central regulator of angiogenesis (formation of new blood vessels from pre-existing vessels) and vasculogenesis (development of embryonic vasculature through an influence on endothelial cell differentiation and organization). Diseases that are characterized by abnormal angiogenesis and vasculogenesis, such as some cancers and some ocular diseases, may show abnormal production of VEGF. Thus, control of VEGF function may be one means to control or treat these diseases. Sirolimus has also been used in the prevention of smooth muscle hyperplasia after coronary stent surgery. The use of sirolimus and ascomycin as specific medicaments for treatment of ocular disease has been disclosed in co-pending U.S. Patent application Serial No. 10/631,143.

Everolimus, also known as RAD-001, SCZ RAD, Certican□ (Novartis, Basel Switzerland), is an analog,of sirolimus but is a new and distinct chemical entity. It is an oral immunosuppressant that inhibits growth factor-induced cell proliferation and thus reduces acute organ rejection, vasculopathy, and/or the proliferation of smooth muscle cells in the innermost wall of grafts that restricts blood supply.

It will be appreciated that the invention encompasses the use of macrolides in addition to those previously described. These include, for example, the known antibiotics ascomycin, erythromycin and its derivatives such as azithromycin and clarithromycin, clindamycin, lincomycin, dirithromycin, josamycin, spiramycin, diacetyl-midecamycin, troleandomycin, tylosin, and roxithromycin, and other macrolides such as biolimus, ABT-578 (methylrapamycin); macrolide derivatives such as temsirolimus (CCI-779, Wyeth) and AP23573 (Ariad) (both rapamycin derivatives). The invention also includes new macrolide antibiotic scaffolds and derivatives in development, including but not limited to the ketolides ABT-773 and telithromycin as described by Schonfeld and Kirst (Eds.) in Macrolide Antibiotics, Birkhauser, Basel Switzerland (2002); macrolides derived from leucomycins, as described in U.S. Patent Nos. 6,436,906; 6,440,942; and 6,462,026 assigned to Enanta Pharmaceuticals (Watertown MA); and lincosamides.

Any of the above-described macrolides may be used in the invention. In one embodiment, the total macrolide concentration ranges from less than 1 ng/ml to about 10 mg/ml. In another embodiment, the total macrolide concentration ranges from about 1 ng/ml to about 1 mg/ml. In another embodiment, the total macrolide concentration is below 5 mg/ml.

It is contemplated that the invention includes the administration of a macrolide or macrolide analog, with neuro-stimulatory and/or neuro-protective activity. In a preferred embodiment, the macrolide or macrolide analog has neuro-stimulatory and/or neuro-protective activity. In one embodiment, the macrolide or macrolide analog is co-administered with a compound having neuro-stimulatory or nuero-protective activity. In a further embodiment of the invention; macrolides can be administered in a concentration ranging from about 20 µg/ml to about 200 µg/ml. Formulations and doses of macrolides are described in U.S. Patent Application Serial Nos. 10/667,161 and 10/752,124, each of which is expressly incorporated by reference herein in its entirety. In an embodiment, a concentration of macrolide antibiotic may range from about 0.1 % to about 10% in a topical ocular formulation for administering to patients with retinitis pigmentosa. In another embodiment, concentrations of macrolide antibiotic up to about 2%, up to about 5%, up to about 10%, or exceeding 10% are formulated for topical administration when the compound(s) is bound to a matrix or polymer which slowly releases the compound(s) over time while not exceeding an intraocular concentration of 40 µg/ml.

Specific macrolide analogues accelerate nerve regeneration and functional recovery, as disclosed in Revill et al., J. Pharmacol. Exp. Therap. (2002) 302; 1278, which is expressly incorporated by reference herein in its entirety. For example, genetically engineered 13- and 15-desmethoxy analogs of ascomycin, examples of macrolide analogs, that contain hydrogen, methyl, or ethyl instead of methoxy at either the 13-, the 15-, or both the 13- and 15-positions enhanced neurite outgrowth in cultured SH-SY5Y neuroblastoma cells at concentrations of 1 mg/kg/day and 5 mg/kg/day, with nerve growth factor (NGF) at a concentration of 10 ng/ml. The ascomycin analog 13-desmethoxy-13-methyl-18 hydroxy (13-Me-18-OH), at concentrations of 1 mg/kg/day and 5 mg/kg/day, was demonstrated to accelerate nerve regeneration and lead to full functional recovery (walking) in a rat sciatic nerve crush model.

The combination of a macrolide and another neurostimulatory or neuroprotective factor(s) such as neurotrophins and/or neuropoietins is used in one embodiment.

Neurotrophins are a family of polypeptides that enhance survival of nervous tissue by maintenance, growth, differentiation, etc. They stimulate the growth of sympathetic and sensory nerve cells in both the central and peripheral nervous system. All neurotrophins have six conserved cysteine residues and share a 55% amino acid sequence identity. Some are in a pro-neurotrophin form and are cleaved to produce a mature form. Examples of neurotrophins include nerve growth factor-β (NGFβ), brain-derived neurotrophic factor (BDNF), neurotrophin 3 (NT-3), neurotrophin 4 (NT-4), neurotrophin 6 (NT-6). These are available commercially, for example, from Sigma-Aldrich (St. Louis MO); Axxora (San Diego CA) mouse 2.5S and 7S components NGFβ, human recombinant β-NGF and pro-β-NGF. A further example of neurotrophins are products of the Neural Regeneration Protein (NRP) gene.

Different neuron types require different neurotrophins, depending upon their receptor expression. All neurotrophins are capable of binding to p75 neurotrophin growth factor receptors, which are low affinity receptors. Specific neurotrophins and mature neurotrophins bind to different tyrosine kinase (trk) receptors, which are higher affinity receptors than p75 receptors. Tyrosine kinase receptors include types A (trkA), B (trkB), and C (trkC).

NGFβ is a specific ligand for the trkA receptor and signals through trkA. It also signals through the low affinity p75 receptors. NGFβ is a secreted protein that helps to develop and maintain the sympathetic nervous system, affecting sensory, pain, and sympathetic targets. It is required for survival of small, peptide-expressing neurons that express the trkA receptor and that project into the superficial laminae of the dorsal horn (i.e., putative nociceptive neurons).

BDNF signals through trkB, in addition to the low affinity p75 receptors. It is Ca2+ dependent and may control synaptic transmission and long term synaptic plasticity, affecting sensory and motor targets. It enhances survival and differentiation of several classes of neurons in vitro, including neural crest and placode-derived sensory neurons, dopaminergic neurons in the substantia nigra, basal forebrain cholinergic neurons, hippocampal neurons, and retinal ganglial cells. BDNF is expressed within peripheral ganglia and is not restricted to neuronal target fields, so that it may have paracrine or autocrine actions on neurons as well as non-neuronal cells.

Neurotrophin-3 (NT-3) is part of the family of neurotrophic factors that control survival and differentiation of mammalian neurons. NT-3 is closely related to NGFβ and BDNF. The mature NT-3 peptide is identical in all mammals examined including human, pig, rat and mouse. NT-3 preferentially signals through trkC, over trkA and trkB receptors, and also utilizes the low affinity p75 receptors. It functions at the neuromuscular junction, affecting large sensory and motor targets and regulating neurotransmitter release at neuromuscular synapses. It may be involved in maintenance of the adult nervous system, and affect development of neurons in the embryo when it is expressed in human placenta.

Neurotrophin 4 (NT-4, synonymous with NT-5) belongs to the NGF-β family and is a survival factor for peripheral sensory sympathetic neurons. NT-4 levels are highest in the prostate, with lower levels in thymus, placenta, and skeletal muscle. NT-4 is also expressed in embryonic and adult tissues. It signals through trkB in addition to low affinity p75 receptors, affecting sympathetic, sensory, and motor targets. Neurotrophin-6 has also been reported.

Ciliary neurotrophic factor (CNTF) is expressed in glial cells within the central and peripheral nervous systems. It stimulates gene expression, cell survival, or differentiation in a variety of neuronal cell types such as sensory, sympathetic, ciliary, and motor neurons. CNTF itself lacks a classical signal peptide sequence of a secreted protein, but is thought to convey its cytoprotective effects after release from adult glial cells by some mechanism induced by injury. In addition to its neuronal actions, CNTF also acts on non-neuronal cells such as glia, hepatocytes, skeletal muscle, embryonic stem cells, and bone marrow stromal cells.

Glial cell derived neurotrophic factor (GDNF) is a 20 kD glycosylated polypeptide that exists as a homodimer. It stimulates the growth of dopaminergic neurons and autonomic motor neurons.

Neuropoietic factors may be used in addition to, or in place of, neurotrophic factors. Neuropoietic factors regulate the properties of cells both in the peripheral and central nervous systems, and both during development and in the mature nervous system. They regulate neuronal phenotype (neurotransmitter) and differentiation of neuronal precursor cells in peripheral and spinal cord neurons. They also regulate cell survival, and development of astrocytes and oligodendrocytes. Neuropoietic factors are also trauma factors in rescuing sensory and motor neurons from axotomy-induced cell death. They show temporal and spatial specific expression patterns, and have specific roles in neural development and repair.

Neuropoietic factors include some cytokines, different from cytokines associated with apoptosis-induced neurodegenerative processes, and hematopoietic factors that fulfill criteria for demonstrating a role in neuronal differentiation and survival. They include leukemia inhibitory factor (LIF), oncostatin M, growth-promoting activity, and cardiotrophin 1. All of these factors activate a subfamily of class I cytokine receptors, the interleukin-6 receptor family.

Any of the above-described neurotrophins and/or neuropoietic factors may be used in the invention. In one embodiment, the total concentration of neurotrophins and/or neuropoietic factors ranges from about 1 pM to about 100 pM. In another embodiment, the total concentration of neurotrophins and/or neuropoietic factors ranges from about 0.01 nM. to about 1 M. In another embodiment, the total concentration of neurotrophins and/or neuropoietic factors is below 1 nM. The neurotrophin(s) and/or neuropoietic factor(s) may be used singly or in combination.

The addition of a macrolide, and/or macrolide analog, with neurostimulatory and/or neuroprotective activity, neurotrophin and/or a neuropoietic factor, alone or in combination, in an ocular formulation, provides beneficial results in enhanced corneal sensation, nerve regeneration, protection including neural protection, and/or re-enervation. In embodiments where a macrolide is present, the composition also reduces post ocular surgical scarring, and provides anti-inflammatory and anti-infective properties. It will be appreciated that various embodiments are contemplated. As one example, a macrolide or macrolide analog, may be used without a neurotrophin or neuropoietic factor. As another example, a neurotrophin or neuropoietic factor or any other neuro-stimulatory factor or factors may be used alone. As another example, other agents may be included in the composition. Examples of these agents include, but are not limited to, steroids, non-steroidal anti-inflammatory agents (NSAIDS), antibiotics, antioxidants, anti-proliferative agents, anti-cell migration agents, anti-angiogenic agents, vitamins, minerals, growth factors and/or cytokines.

Steroids for ocular administration include, but are not limited to, triamcinolone (Aristocort®; Kenalog®), betamethasone (Celestone®), budesonide, cortisone, dexamethasone (Decadron-LA®; Decadron® phosphate; Maxidex® and Tobradex® (Alcon)), hydrocortisone, methylprednisolone (Depo-Medrol®, Solu-Medrol®), prednisolone (prednisolone acetate, e.g., Pred Forte® (Allergan); Econopred and Econopred Plus® (Alcon); AK-Tate® (Akorn); Pred Mild® (Allergan); prednisone sodium phosphate (Inflamase Mild and Inflamase Forte® (Ciba); Metreton® (Schering); AK-Pred® (Akorn)), fluorometholone (fluorometholone acetate (Flarex® (Alcon); Eflone®), fluorometholone alcohol (FML® and FML-Mild®, (Allergan); FluorOP®)), rimexolone (Vexol® (Alcon)), medrysone alcohol (HMS® (Allergan)); lotoprednol etabonate (Lotemax® and Alrex® (Bausch & Lomb), 11-desoxcortisol, and anecortave acetate (Alcon)).

The steroid concentration used with a particular formulation will depend upon the particular steroid that is used. For example, triamcinolone acetonide (9α-fluoro-1 1 13, 16a, 17, 21 tetra hydroxy-pregna-1,4-diene-3,20-dione cyclic 16,17-acetal with acetone (C24H31F06)) Kenacort®, Kenalog® (Bristol-Myers Squibb, Princeton NJ) may be administered at a therapeutic dose in the range of about 4 mg to about 8 mg, for example, by intravitreous injection. In comparison, anecortave acetate, a steroid with less potential to cause an increase in intraocular pressure than triamcinolone but not used inside the eye, may be administered at a dose of about 0.1 mg/ml to about 30 mg/ml.

Antibiotics include, but are not limited to, doxycycline (4-(dimethylamino)-1,4,4a,5,5a,6,11,12a-octahydro-3,5,10,12,12a-pentahydroxy-6-methyl-1, 11-dioxo-2-naphthacenecarboxamide monohydrate, C22H24N2O8·H2O), aminoglycosides (e.g., streptomycin, amikacin, gentamicin, tobramycin), cephalosporins (e.g., beta lactams including penicillin), tetracyclines, acyclorvir, amantadine, polymyxin B, amphtotericin B, amoxiciflin, ampicillin, atovaquone, azithromycin, azithromycin, bacitracin, cefazolin, cefepime, cefotaxime, cefotetan, cefpodoxime, ceftazidime, ceftizoxime, ceftriaxone, cefuroxime, cephalexin, chloramphenicol,clotimazole, ciprofloxacin, clarithromycin, clindamycin, dapsone, dicloxacillin, erythromycin, fluconazole, foscarnet, ganciclovir, gatifloxacin, griseofulvin, isoniazid, itraconazole, ketoconazole, metronidazole, nafcillin, neomycin, nitrofurantoin, nystatin, pentamidine, rifampin, rifamycin, valacyclovir, vancomycin, etc.

Anti-proliferative agents include, but are not limited to, carboplatin, 5-fluorouracil (5-FU), thiotepa, etoposide (VP-16), doxorubicin, ifosphophamide, cyclophosphamide, etc.

Anti-prostaglandins include, but are not limited to, indomethacin, ketorolac tromethamine 0.5% ((±)-5-benzoyl-2,3-dihydro-1H-pyrrolizine-1-carboxylic acid, compound with 2-amino-2-(hydroxymethyl)-1,3-propanediol (1:1) (ACULAR® Allegan, Irvine CA), OCUFEN® (flurbiprofen sodium 0.03%), meclofenamate, flurbiprofen, and compounds in the pyrrolo-pyrrole group of non-steroidal anti-inflammatory drugs. As one example, antiprostaglandins such as flurbiprofen may be administered at a concentration in the range of about 0.001%w/v to about 0.5%w/v. As an example, OCUFEN® (flurbiprofen sodium 0.03% (Allergan), sodium (±)-2-(2-fluoro-4-biphenylyl)-propionate dihydrate) 0.03% may be administered at a concentration ranging from about 0.003% w/v to about 0.3% w/v. Anti-prostaglandins other than flurbiprofen may be included. For example, ACUALR® may be administered at a concentration ranging from about 0.003%w/v to about 0.3%w/v. In one embodiment, the concentration of ACULAR® is about 0.03% w/v.

A matrix metalloproteinase inhibitor may be added. These include, but are not limited to, doxycycline, demeclocycline, minocycline, oxytetracycline, lymecycline, a chemically modified tetracycline, TIMP-1, TIMP-2, TIMP-3, TIMP-4, MMP1, MMP2, MMP3, Batimastat, Marimastat, BAY 12-9566, or KB-R7785. Chemically modified tetracyclines (CMT) include demeclocycline, minocyciine, oxytetracycline and like compounds that inhibit the synthesis of MMP-8 and MMP-9. These include CMT such as CMT-315, CMT-3, CMT-8, and CMT-308; 6-demethyl-6-deoxy-4-dedimethylaminotetracylcine (COL-3), and others, for example, as described by Liu et a/., A Chemically Modified Tetracycline (CMT-3) Is a New Antifungal Agent in Antimicrobial Agents Chemother. 2002 May; 46:1447; Seftor et al., Targeting the Tumor Microenvironment with Chemically Modified Tetracyclines: Inhibition of Laminin 5γ2 Chain Promigratory Fragments and Vasculogenic Mimicry 2002 November; 1: 1173, which are expressly incorporated by reference herein.

Inhibitors of matrix metalloproteinases include naturally occurring proteins such as TIMP-1 that specifically inhibit matrix metalloproteinases, and synthetic metalloproteinase inhibitors such as Batimastat (BB-94) and marimastat (BB-2516) which potently and specifically inhibit metalloproteinase production. These inhibitors degrade the extracellular matrix, promoting tumor invasion and metastasis, but also regulate host defense mechanisms and normal cell function. Selective inhibition is expected to inhibit reactions leading to neovascularization in the inventive formulations and methods. Such metalloproteinase inhibitors are also included in the invention. Among the twenty-four MMPs described, eight have been identified in the cornea, i.e., collagenase I and III (MMP-1 and MMP-13), gelatinase A and B (MMP-2 and -9), stromelysin (MMP-3), matrilysin (MMP-7) and membrane type MMP (MMP-14).

Anti-angiogenesis agents include, but are not limited to, antibodies to vascular endothelial growth factor (VEGF) such as bevacizumab (AVASTIN®) and rhuFAb V2 (ranibizumab) (Genentech), and other anti-VEGF compounds; pigment epithelium derived factor(s) (PEDF); CELEBREX®; VIOXX®; interferon alpha; interleukin-12 (IL-12); thalidomide and derivatives such as REVIMID™(CC-5013) (Celgene Corporation); squalamine; endostatin; angiostatin; the ribozyme inhibitor ANGIOZYME® (Sirna Therapeutics); multifunctional antiangiogenic agents such as NEOVASTAT® (AE-941) (Aeterna Laboratories, Quebec City, Canada); etc., as known to one skilled in the art. For example formulations of the invention may be injected with anti-angiogenic agents designed to block the actions of VEGF on endothelial cells that can be employed in the method of the invention are: (a) Lucentis^{®} developed by Genentech; and (b) Macugen^{®} developed by Eyetech Pharmaceuticals. Lucentis^{®} and Macugen^{®} are compounds that are injected into the vitreous and are potent anti-angiogenic compounds. In an alterative embodiment, the pharmaceutical formulation of the invention will comprise a formulation of the invention as described and an anti-angiogenic agent such as Lucentis^{®} or Macugen^{®}.

Lucentis^{®} (ranibizumab), formerly known as rhuFab V2 or AMD-Fab is a humanized, therapeutic anti-VEGF (vascular endothelial growth factor) antibody fragment developed at Genentech to bind and inhibit VEGF, a protein that plays a critical role in angiogenesis (the formation of new blood vessels). Lucentis^{®} is designed to block new blood vessel growth and reduce leakage, which are thought to lead to wet AMD disease progression. When administered in conjunction with pharmaceutical formulations prepared according to the present invention Lucentis^{®} should be administered in either about 300 or about 500 microgram doses for four doses.

Macugen^{®} (pegaptanib sodium, anti-VEGF aptamer or EYE001) developed by Eyetech Pharmaceuticals, consists of a synthetic fragment of genetic material that specifically binds to the VEGF molecule and blocks it from stimulating the receptor on the surface of endothelial cells. When administered in conjunction with pharmaceutical formulations prepared according to the present invention Macugen^{®} should be administered in a dose ranging from either about 0.3 mg to about 3.0 mg every four or six weeks.

In another aspect of the invention, pharmaceutical formulations prepared according to the present invention may be prepared in combination with a glucocorticoid (e.g. prednisolone, prednisone), an oestrogen (e.g. oestrodiol), an androgen (e.g. testosterone) retinoic acid derivatives (e.g. 9-cis-retinoic acid, 13-trans-retinoic acid, all-trans retinoic acid), a vitamin D derivative (e.g. calcipotriol, calcipotriene), a non-steroidal anti-inflammatory agent, a vitamin D derivative, an anti-infective agent, a protein kinase C inhibitor, a MAP kinase inhibitor, an anti-apoptotic agent, a growth factor, a nutrient vitamin, an unsaturated fatty acid, and/or ocular anti-infective agents, for the treatment of the ophthalmic disorders set forth herein. In still other embodiments of the invention, a mixture of these agents may be used.

Ocular anti-infective agents as described herein include, but are not limited to, penicillins (ampicillin, aziocillin, carbenicillin, dicloxacillin, methicillin, nafcillin, oxacillin, penicillin G, piperacillin, and ticarcillin), cephalosporins (cefamandole, cefazolin, cefotaxime, cefsulodin, ceftazidime, ceftriaxone, cephalothin, and moxalactam), aminoglycosides (amikacin, gentamicin, netilmicin, tobramycin, and neomycin), miscellaneous agents such as aztreonam, bacitracin, ciprofloxacin, clindamycin, chloramphenicol, cotrimoxazole, fusidic acid, imipenem, metronidazole, teicoplanin, and vancomycin), antifungals (amphotericin B, clotrimazole, econazole, fluconazole, flucytosine, itraconazole, ketoconazole, miconazole, natamycin, oxiconazole, and terconazole), antivirals (acyclovir, ethyldeoxyuridine, foscarnet, ganciclovir, idoxuridine, trifluridine, vidarabine, and (S)-1-(3-dydroxy-2-phospho-nyluethoxypropyl) cytosine (HPMPC)), antineoplastic agents (cell cycle (phase) nonspecific agents such as alkylating agents (chlorambucil, cyclophosphamide, mechlorethamine, melphalan, and busulfan), anthracycline antibiotics (doxorubicin, daunomycin, and dactinomycin), cisplatin, and nitrosoureas), antimetabolites such as antipyrimidines (cytarabine, fluorouracil and azacytidine), antifolates (methotrexate), antipurines (mercaptopurine and thioguanine), bleomycin, vinca alkaloids (vincrisine and vinblastine), podophylotoxins (etoposide (VP-16)), and nitrosoureas (carmustine, (BCNU)), immunosuppressant agents such as cyclosporin A and FK506, and anti-inflammatory or suppressive factors (inhibitors), and inhibitors of proteolytic enzymes such as plasminogen activator inhibitors.

Other agents may also be added, such as NIDS, vitamins, minerals, cytokines, growth factors, etc. Examples of the above include, but are not limited to, colchicine, naproxen sodium (ANAPROX® and ANAPROX DS®, (Roche); flurbiprofen (ANSAID®, Pharmacia Pfizer); diclofenac sodium and misoprostil (ARTHROTEC®, Searle Monsanto); valdecoxib (BEXTRA®, Pfizer); diclofenac potassium (CATAFLAM®, Novartis); celecoxib (CELEBREX®, Searle Monsanto); sulindac (CLINORIL®, Merck); oxaprozin (DAYPRO®, Pharmacia Pfizer); salsalate (DISALCID®, 3M); salicylate (DOLOBID®, Merck); naproxen sodium (EC NAPROSYN®, Roche); piroxicam (FELDENE®, Pfizer); indomethacin (INDOCIN®, Merck); etodolac (LODINE®, Wyeth); meloxicam (MOBIC®, Boehringer Ingelheim); ibuprofen (MOTRIN®, Pharmacia Pfizer); naproxen (NAPRELAN®, Elan); naproxen (NAPROSYN®, Roche); ketoprofen (ORUDIS®, ORUVAIL®, Wyeth); nabumetone (RELAFEN®, SmithKline); tolmetin sodium (TOLECTIN®, McNeil); choline magnesium trisalicylate (TRILISATE®, Purdue Fredrick); rofecoxib (VIOXX®, Merck), vitamins A, B (thiamine), B6 (pyridoxine), B12 (cobalamine), C (ascorbic acid), D1, D2 (ergocalciferol), D3 (cholcalciferol), E, K (phytonadione), K1 (phytylmenaquinone), K2 (multiprenylmenaquinone), carotenoids such as lutein and zeaxanthin; macrominerals and trace minerals including, but not limited to, calcium, magnesium, iron, iodine, zinc, copper, chromium, selenium, manganese, molybdenum, fluoride, boron, etc. Commercially available supplements are also included such as high potency zinc (commercially available as OCUVITE® PRESERVISION®, Bausch & Lomb, Rochester NY), or high potency antioxidants (zinc, lutein, zeaxanthin) (commercially available as ICAPS® Dietary Supplement, Alcon, Fort Worth TX).

It will be appreciated that the above agents include pharmaceutically acceptable salts and derivatives thereof (e.g., sodium, potassium, bicarbonate, sulfate, etc). It will also be appreciated that the above lists are representative only and are not exclusive. The indications, effective doses, formulations (including buffers, salts, and other excipients), contraindications, vendors, etc. of each of the above are known to one skilled in the art.

The duration over which any of the formulations of the invention will dwell in the ocular environment will depend, *inter alia,* on such factors as the pharmacological properties of the compounds employed in the formulation, the concentration of the compound employed, the bioavailability of the compound, the disease to be treated the mode of administration and the preferred longevity of the treatment. Where that balance is struck will often depend on the longevity of the effect required in the eye and the ailment for which the composition is administered. Formulations prepared according to the invention may have dwell times from hours to many months and possibly years, although the latter time period requires special delivery systems to attain such a duration. Some illustrative forms of such delivery systems are disclosed below. In one embodiment, the formulations described herein will have a dwell time (ie duration in the eye) of hours (i.e. 1 to 24 hours), days (i.e. 1, 2, 3, 4, 5, 6 or 7 days) or weeks (i.e. 1, 2, 3, 4 weeks). Alternatively, the formulation will have a dwell time of at least a few months such as, 1 month, 2 months, 3 months, with dwell times of greater than 4, 5, 6, 7 to 12 months being achievable.

The precise formulation used in the pharmaceutical formulation of the present invention will vary according to a wide range of commercial and scientific criteria. The above formulation of the invention described above may contain other agents. For example the formulations of the invention may be prepared using a physiological saline solution as a vehicle. The pH of the formulation may be maintained at a substantially neutral pH (for example, about 7.4, in the range of about 6.5 to about 7.4, etc.) with an appropriate buffer system as known to one skilled in the art (for example, acetate buffers, citrate buffers, phosphate buffers, borate buffers).

The formulation may additionally include at least a pharmaceutically acceptable additive (such as a diluent, carrier, adjunct, excipient or non-toxic, non-therapeutic, non-immunogenic stabilizers and the like). The pharmaceutically acceptable additive should be ophthalmologically acceptable, and should not leave any vision impairing residue in the eye. Any pharmaceutically acceptable additive used in the formulation may be suited to the delivery of said pharmaceutical formulation as an intravitreal depot injection.

Any diluent used in the preparation of the pharmaceutically acceptable formulation may be selected so as not to unduly affect the biological activity of the formulation. Examples of such diluents which are useful for injectable formulations are water, the various saline, organic or inorganic salt solutions, Ringer's solution, dextrose solution, and Hank's solution.

In addition, the pharmaceutical formulation may include additives such as other buffers, diluents, carriers, adjuvants or excipients. Any pharmacologically acceptable buffer suitable for application to the eye may be used, e.g., tris or phosphate buffers. Other agents may be employed in the formulation for a variety of purposes. For example, buffering agents, preservatives, co-solvents, surfactants, oils, humectants, emollients, chelating agents, stabilizers or antioxidants may be employed. Water soluble preservatives which may be employed include, but are not limited to, benzalkonium chloride, chlorobutanol, thimerosal, sodium bisulfate, phenylmercuric acetate, phenylmercuric nitrate, ethyl alcohol, methylparaben, polyvinyl alcohol, benzyl alcohol and phenylethyl alcohol. A surfactant may be Tween 80. Other vehicles that may be used include, but are not limited to, polyvinyl alcohol, povidone, hydroxypropyl methyl cellulose, poloxamers, carboxymethyl cellulose, hydroxyethyl cellulose, purified water, etc. Tonicity adjustors may be included, for example, sodium chloride, potassium chloride, mannitol, glycerin, etc. Antioxidants include, but are not limited to, sodium metabisulfite, sodium thiosulfate, acetylcysteine, butylated hydroxyanisole, butylated hydroxytoluene, etc. The indications, effective doses, formulations, contraindicatons, vendors etc, of the compounds in the formulations are available or are known to one skilled in the art.

These agents may be present in individual amounts of from about 0.001% to about 5% by weight and, in one embodiment, about 0.01% to about 2%. Suitable water soluble buffering agents that may be employed are sodium carbonate, sodium borate, sodium phosphate, sodium acetate, sodium bicarbonate, etc., as known to one skilled in the art for the desired route of administration. These agents may be present in amounts sufficient to maintain a pH of the system of between about 2 to about 9 and, in one embodiment, about 4 to about 8. As such the buffering agent may be as much as about 5% on a weight to weight basis of the total formulation: Electrolytes such as, but not limited to, sodium chloride and potassium chloride may also be included in the formulation.

The composition may be formulated as a slow, extended, or time release formulation, a carrier formulation such as microspheres, microcapsules, liposomes, etc., as known to one skilled in the art. Any of the above-mentioned delayed release delivery systems may be administered topically, intraocularly, subconjunctivally, or by implant to result in sustained release of the agent over a period of time. The formulation may be in the form of a vehicle, such as a micro- or macro-capsule or matrix of biocompatible polymers such as polycaprolactone, polyglycolic acid, polylactic acid, polyanhydrides, polylactide-co-glycolides, polyamino acids, polyethylene oxide, acrylic terminated polyethylene oxide, polyamides, polyethylenes, polyacrylonitriles, polyphosphazenes, poly(ortho esters), sucrose acetate isobutyrate (SAIB), and other polymers such as those disclosed in U.S. Patent Nos. 6,667,371; 6,613,355; 6,596,296; 6,413,536; 5,968,543; 4,079,038; 4,093,709; 4,131,648; 4,138,344; 4,180,646; 4,304,767; 4,946,931, each of which is expressly incorporated by reference herein in its entirety, or lipids that may be formulated as microspheres or liposomes.

A microscopic or macroscopic formulation may be administered topically or through a needle, or may be implanted.

Delayed or extended release properties may be provided through various formulations of the vehicle (coated or uncoated microsphere, coated or uncoated capsule, lipid or polymer components, unilamellar or multilamellar structure, and combinations of the above, etc.). The formulation and loading of microspheres, microcapsules, liposomes, etc. and their ocular implantation are standard techniques known by one skilled in the art, for example, the use a ganciclovir sustained-release implant to treat cytomegalovirus retinitis, disclosed in Vitreoretinal Surgical Techniques, Peyman et al., Eds. (Martin Dunitz, London 2001, chapter 45); Handbook of Pharmaceutical Controlled Release Technology, Wise, Ed. (Marcel Dekker, New York 2000), the relevant sections of which are incorporated by reference herein in their entirety. For example, a sustained release intraocular implant may be inserted through the pars plana for implantation in the vitreous cavity. An intraocular injection may be into the vitreous (intravitreal), or under the conjunctiva (subconjunctival), or behind the eye (retrobulbar), or under the Capsule of Tenon (sub-Tenon), and may be in a depot form. The composition may be administered via a contact lens applied to the exterior surface of an eye, with the composition incorporated into the lens material (e.g., at manufacture, or contained in a lens solution). The composition may be administered via an intraocular lens (IOL) that is implanted in the eye. Implantable lenses include any IOL used to replace a patient's diseased lens following cataract surgery, including but not limited to those manufactured by Bausch and Lomb (Rochester NY), Alcon (Fort Worth TX), Allergan (Irvine CA), and Advanced Medical Optics (Santa Ana CA). When the lens is implanted within the lens capsule, the composition provides the desired effect to the eye. Concentrations suitable for implants (lenses and other types) and by contact lens administration may vary, as will be appreciated by one skilled in the art. For example, an implant may be loaded with a high amount of agent, but formulated or regulated so that a required concentration within the above-described ranges is sustainedly released (e.g., slow release formulation).

In various embodiments, the composition is administered up to four times a day. In embodiments where the composition is administered after surgery, administration may commence following surgery on the same day, or the day after surgery, or a few days after surgery, or any time after surgery. The composition may be self-administered or administered by another, for example, if visual acuity is poor, or if the patient is uncomfortable with self-administration. The patient is periodically evaluated (e.g., daily, every other day, etc.) using assessment methods known to one skilled in the art. In embodiments where the composition is used to assess corneal sensation, these include assessment of corneal clarity, corneal sensation (e.g., using a Cochet-Bonnet filament-type aesthesiometer), corneal enervation, etc. In embodiments where the composition is used to enhance ocular neuroprotection and/or neurostimulation, these may include one or more of the following assessments: retinal ganglial cell viability, quantitation of ocular glutamate levels, visual field and visual acuity determinations, assessment of visual evoked potential (VEP) to evaluate visual neural pathways via electrode measurement of brain electrical activity while watching a moving pattern on a video monitor, electroretinogram (ERG) to evaluate the ocular electrical responses to a flash of light using an electrode placed on the surface of the eye (e.g., cornea), electrooculargram (EOG), critical flicker fusion (CFF) test that measures a sensitivity threshold to provide information about the temporal responsiveness of visual pathways, etc. These assessments are known to one skilled in the art.

Other variations or embodiments of the invention will also be apparent to one of ordinary skill in the art from the above description. As one example, the invention may be used to facilitate growth of transplanted neuronal cells, either mature or immature, and/or stem cells in the eye or brain. As another example, other ocular routes of administration and injection sites and forms are also contemplated. As another example, the invention may be used in patients who have experienced ocular trauma, ischemia, inflammation, etc. Thus, the forgoing embodiments are not to be construed as limiting the scope of this invention.

### Non-limiting illustration of the invention

Further features of the present invention are more fully described in the following non-limiting Examples. This description is included solely for the purposes of exemplifying the present invention. It should not be understood as a restriction on the broad description of the invention as set out above.

To generate damage to retinal sensory or ganglion cells, anesthetized rabbits are subjected to (a) a severe crush injury of the optic nerve, (b) or may be treated to induce increased intraocular pressure, (c) or may be treated to induce retinal ischemic/reperfusion injury, (d) or may be subject to other methods known to one skilled in the art.

A severe crush injury of the optic nerve may be caused in the following manner - as an example of one test -- after excising the conjunctiva and exposing the optic nerve with the aid of a binocular operating microscope with care not to interfere with the blood supply, the nerve can be mechanically crushed for a defined period using forceps or other instruments, as described in Schori et al., (2001) PNAS 98:3398, which is expressly incorporated by reference herein in its entirety.

Intraocular pressure changes may be induced when rabbits are treated to an intraocular pressure greater than 17 mm Hg. This may be done by negative pressure applied to a corneoscleral ring fixed to the sclera and connected to a vacuum source, as known to one skilled in the art. This may also be done by positive pressure applied through a cannula connected to the interior chamber. This may also be done by blocking aqueous outflow using 80-120 applications of blue-green argon laser as described in Bakalash et al., Investigative Ophthalmology & Visual Science 44: 3374 (2003), which is expressly incorporated by reference herein.

Retinal ischemic/reperfusion injury may be caused by closing the central and choroidal arteries. Deprivation of blood flow to the retina results in ischemia due to lack of oxygen and nutrients, while reperfusion results in free radical injury; this type of ischemia/reperfusion injury is known to one skilled in the art.

A combination of the macrolides and/or macrolide analogues, with neuro-stimulator and/or neuro-protective activity, neurotrophins, and neuropoietic factors in different combinations of agent, dose, route of administration, intervals, etc. as described herein is then administered as previously described.

Assessment of retinal damage in control' and treated animals is by applying dextran tetramethylrhodamine, a hydrophilic neurotracer (Molecular Probes, Eugene OR) into the intraorbital portion of the optic nerve, with only functional axons capable of dye uptake. Rabbits are sacrificed twenty-four hours after dye administration, retinas excised, wholemounted, and preserved in 4% paraformaldehyde. Retinal ganglial cells are counted under 800x magnification using a fluorescence microscope. Four fields from each retina are counted with the same diameter and located the same distance from the optic disc. Eyes from untreated rabbits are used as controls.

Other dyes or markers for viable ganglion cells can be introduced and the number of cells can be counted in treated groups versus control groups. In addition, factors other than dye uptake can be used as an indicator of neuroprotection and/or neurostimulation. These factors include retinal ganglial and/or sensory cell morphology from treated versus control groups, assays of cellular function, conductivity, etc. Conversely, apoptosis may be assayed in retinal ganglial and/or sensory cells from treated versus control groups. For example, Annexin V binding is known by one skilled in the art as an indirect indicator of apoptosis, and binding can be assayed in treated versus control cells. A clonagenic assay is known by one skilled in the art as a direct indicator of apoptosis, and can be performed with the results compared from both treated and control cells.

Modifications of the above-described modes of carrying out the various embodiments of this invention will be apparent to those skilled in the art based on the above teachings related to the disclosed invention. The above embodiments of the invention are merely exemplary and should not be construed to be in any way limiting.

In a further example, 20 human patients of at least 21 years of age and to undergo bilateral LASIK for the treatment of myopia with or without astigmatism can be treated with an embodiment of the method and composition of the invention. In one embodiment, each patient must have a difference in refractive correction between both eyes no greater than 2D sphere and 1D cylinder.

In such an example, eyes are assigned as control eyes and study eyes. In both eye types, the hinge position of the LASIK flap is in the same position. Each set of eyes undergoes the LASIK procedure.

At the completion of LASIK, a qualified practitioner, preferably a surgeon, administers one drop of Ciloxan and one drop of cyclosporine onto the corneal surface of the study eye. This administration is repeated four times each day for Ciloxan, and twice daily for cyclosporine, until evaluation 3 months after surgery. In the most preferred example, the cyclosporine drops are administered substantially 12 hours apart. Cyclosporine is not administered to the control eye. Ciloxan is administered by a qualified practitioner, preferably a surgeon, at the completion of LASIK. This administration is repeated four times each day until evaluation of the control eye, 3 months after surgery.

In one embodiment, patients will be evaluated pre-treatment, immediately post-operative, 1 day post-operative, 1 week post-operative, 1 month post-operative and 3 months post-operative.

In one embodiment, the parameters for evaluation include but are not limited to: distance of uncorrected vision, including the use of Snellen charts and constant illumination; visual acuity, including distance and best corrected, and further including the use of Snellen charts and constant illumination; slit lamp, including the examination of the eyelid margin, conjunctiva, cornea and anterior segment; manifest reaction; corneal sensitivity, including measured with a Cochet-Bonnet esthesiometer and using measurements taken at nine different areas of each cornea such as, for example, four quadrants of untreated cornea, the peripheral areas of the flap and the centre of the flap; subjective patient questionnaire, including questions relating to ocular moisture, foreign body sensation, burning/stinging, stickiness, blurred vision, pain/soreness, redness, crusting or discharge, light sensitivity, comfort in wind/air conditioning; and verbal or written questioning regarding adverse reactions and/or complications.

The invention will now be described by way of a series of numbered clauses
1. A method of enhancing at least one of ocular neuroprotection and/or neurostimulation in or to the eye of a patient, said method comprising the step of locally administering in a non-systemic manner to the eye of a patient, a composition comprising at least one neuro-protective and/or neuro-stimulatory factor in a pharmaceutically effective formulation.
2. Use of at least one neuro-protective and/or neuro-stimulatory factor for preparation of a medicament for enhancing ocular neuroprotection and/or neurostimulation in or to the eye of a patient.
3. A method according to clause 1 wherein there is also present in the pharmaceutically effective formulation at least one neurotrophin or neuropoietic factor.
4. Use of at least one neuro-protective and/or neuro-stimulatory factor and at least one neurotrophin or neuropoietic factor for preparation of a medicament for enhancing ocular neuroprotection and/or neurostimulation in or to the eye of a patient.
5. An ocular method for ameloirating a post-ocular surgery patient or a trauma patient, comprising the step of: administering to said patient, a composition comprising at least one neuro-protective and/or neuro-stimulatory factor in a pharmaceutically effective formulation adapted for localized non-systemic ocular administration.
6. Use of at least one neuro-protective and/or neuro-stimulatory factor for the preparation of a medicament for the amelioration of a post-ocular surgery patient or a trauma patient.
7. An ocular method comprising administering to a patient after ocular surgery a composition comprising at least one neuro-stimulatory or neuro-protective factor, which encompasses a macrolide and/or macrolide analog with neuro-stimulatory and/or neuro-protective activity, in a pharmaceutically effective concentration and formulation for non-systemic localized ocular administration.
8. Use of at least one neuro-stimulatory or neuro-protective factor, including a macrolide and/or macrolide analog with neuro-stimulatory and/or neuro-protective activity, for the preparation of a medicament for administration to a post-ocular surgery patient.
9. An ocular method comprising administering to a patient prior to ocular surgery a composition comprising at least one neuro-stimulatory or neuro-protective factor, which encompasses a macrolide and/or macrolide analog with neuro-stimulatory and/or neuro-protective activity, in a pharmaceutically effective concentration and formulation for non-systemic localized ocular administration.
10. Use of at least one neuro-stimulatory or neuro-protective factor, including a macrolide and/or macrolide analog with neuro-stimulatory and/or neuro-protective activity, for the preparation of a medicament for administration to a patient prior to ocular surgery.
11. A method for ameliorating a post-ocular surgery patient with ocular scarring, comprising the step of: administering to said patient, a composition comprising at least one neuro-protective and/or neuro-stimulatory factor in a pharmaceutically effective formulation adapted for localized non-systemic ocular administration.
12. Use of at least one neuro-protective and/or neuro-stimulatory factor for the preparation of a medicament for the amelioration of ocular scarring.
13. A method for ameliorating a ocular surgery patient at risk of ocular scarring, comprising the step of: administering to said patient, a composition comprising at least one neuro-protective and/or neuro-stimulatory factor in a pharmaceutically effective formulation adapted for localized non-systemic ocular administration.
14. Use of at least one neuro-protective and/or neuro-stimulatory factor for the preparation of a medicament for prophylaxis of ocular scarring.
15. A method for enhancing ocular nerve regeneration, and/or re-enervation in the eye of a patient, comprising the step of: administering to said patient, a composition comprising at least one neuro-protective and/or neuro-stimulatory factor in a pharmaceutically effective formulation adapted for localized non-systemic ocular administration.
16. Use of at least one neuro-protective and/or neuro-stimulatory factor for the preparation of a medicament for enhancing ocular nerve regeneration, and/or re-enervation in the eye of a patient.
17. A method for partially or completely ameliorating or restoring the loss of corneal sensation in the eye of a patient that occurs following a corneal procedure during which nerves are severed, comprising the step of: administering to said patient, a composition comprising at least one neuro-protective and/or neuro-stimulatory factor in a pharmaceutically effective formulation adapted for localized non-systemic ocular administration.
18. Use of at least one neuro-protective and/or neuro-stimulatory factor for the preparation of a medicament for partially or completely restoring the loss of corneal sensation in the eye of a patient that occurs following a corneal procedure during which nerves are severed.
19. A method for administering a macrolide and/or macrolide analog, with neuro-stimulatory and/or neuro-protective activity, neurotrophin, and/or neuropoietic agent prophylactically to patients having or at risk for developing an ocular neurologic or neurosensory disease, or therapeutically to patients with an ocular neurologic or neurosensory disease, either alone or in conjunction with other therapy.
20. Use of at least one macrolide and/or macrolide analog, with neuro-stimulatory and/or neuro-protective activity, neurotrophin, and/or neuropoietic agent for preparation of a medicament for administration to a patient having or at risk for developing an ocular neurologic or neurosensory disease.
21. A method for ameliorating a patient with an ocular neurologic or neurosensory ailment, comprising the step of: administering to said patient, a composition comprising at least one neuro-protective and/or neuro-stimulatory factor in a pharmaceutically effective formulation adapted for localized non-systemic ocular administration.
22. Use of at least one neuro-protective and/or neuro-stimulatory factor for preparation of a medicament for administration to a patient having an ocular neurologic or neurosensory ailment.
23. A method for prophylaxis of a patient at risk of developing an ocular neurologic or neurosensory ailment, comprising the step of: administering to said patient, a composition comprising at least one neuro-protective and/or neuro-stimulatory factor in a pharmaceutically effective formulation adapted for localized non-systemic ocular administration.
24. Use of at least one neuro-protective and/or neuro-stimulatory factor for preparation of a medicament for administration to a patient at risk of developing an ocular neurologic or neurosensory ailment.
25. A method of prophylaxis of an individual with an ocular neurologic or neurosensory disease in a first eye but not in the second eye of said individual, comprising the steps of: administering to the second eye of said individual a composition comprising at least one neuro-protective and/or neuro-stimulatory factor in a pharmaceutically effective formulation adapted for localized non-systemic ocular administration.
26. Use of at least one neuro-protective and/or neuro-stimulatory factor for preparation of a medicament for administration to a second eye of a patient wherein the patient has an ocular neurologic or neurosensory disease in a first eye but not the second eye.
27. The method of clause 1 wherein the formulation is administered prophylactically to a patient at risk for glaucoma or retinitis pigmentosa.
28. Use of at least one neuro-protective and/or neuro-stimulatory factor for preparation of a medicament for administration prophylactically to a patient at risk for glaucoma or retinitis pigmentosa.
29. The method of clause 1wherein the formulation is administered to a patient with increased intraocular pressure, open angle glaucoma, and/or retinitis pigmentosa.
30. Use of at least one neuro-protective and/or neuro-stimulatory factor for preparation of a medicament for the amelerioration of increased intraocular pressure, open angle glaucoma, and/or retinitis pigmentosa.
31. The method of clause 1 wherein the formulation is administered in conjunction with a current treatment for increased intraocular pressure to result in a synergistic effect.
32. The method of clause 1 wherein the formulation is administered as an extended release formulation.
33. Use of at least one neuro-protective and/or neuro-stimulatory factor for preparation of an extend release medicament for enhancing ocular neuroprotection and/or neurostimulation in or to the eye of a patient.
34. The method of clause 1 wherein the formulation is administered topically, by intraocularly injection, by injection in the lens, on an ocular lens, or via an ocular device.
35.The method of clause 1 wherein the neuro-protective and/or neuro-stimulatory factor comprises at least one neurotrophin selected from nerve growth factor-β (NGFβ), brain-derived neurotrophic factor (BDNF), neurotrophin 3 (NT-3), neurotrophin 4 (NT-4), neurotrophin 6, ciliary neurotrophic factor (CNTF), or glial cell derived neurotrophic factor (GDNF); or, at least one neuropoietic factor selected from leukemia inhibitory factor (LIF), ciliary neurotrophic factor (CNTF), oncostatin M, growth-promoting activity, or cardiotrophin 1.
36. The method of clause 1 wherein the neuro-protective and/or neuro-stimulatory factor comprises at least one neurotrophin selected from nerve growth factor-β (NGFβ), brain-derived neurotrophic factor (BDNF), neurotrophin 3 (NT-3), neurotrophin 4 (NT-4), neurotrophin 6, ciliary neurotrophic factor (CNTF), or glial cell derived neurotrophic factor (GDNF); and, at least one neuropoietic factor selected from leukemia inhibitory' factor (LIF), ciliary neurotrophic factor (CNTF), oncostatin M, growth-promoting activity, or cardiotrophin 1.
37. Use of at least one of nerve growth factor-β (NGFβ), brain-derived neurotrophic factor (BDNF), neurotrophin 3 (NT-3), neurotrophin 4 (NT-4), neurotrophin 6, ciliary neurotrophic factor (CNTF), glial cell derived neurotrophic factor (GDNF), leukemia inhibitory factor (LIF), ciliary neurotrophic factor (CNTF), oncostatin M, growth-promoting activity, or cardiotrophin 1 for preparation of a medicament for enhancing ocular neuroprotection and/or neurostimulation in or to the eye of a patient.
38. The method of clause 3 wherein the neurotrophin is at least one of nerve growth factor-β (NGFβ), brain-derived neurotrophic factor (BDNF), neurotrophin 3 (NT-3), neurotrophin 4 (NT-4), neurotrophin 6, ciliary neurotrophic factor (CNTF), or glial cell derived neurotrophic factor (GDNF), and the neuropoietic factor is at least one of leukemia inhibitory factor (LIF), ciliary neurotrophic factor (CNTF), oncostatin M, growth-promoting activity, or cardiotrophin 1.
39. A method comprising administering to a patient post-ocular surgery, pre-ocular surgery, with ocular scarring, or at risk of developing ocular scarring, a composition comprising at least one neuro-stimulatory or neuro-protective factor, in a pharmaceutically effective concentration and formulation for non-systemic localized ocular administration, wherein the neuro-stimulatory or neuro-protective factor is a macrolide and/or macrolide analog, with neuro-stimulatory and/or neuro-protective activity, and wherein the viability and/or activity of retinal sensory or ganglion cells is increased over viability and/or activity of the cells in the absence of the macrolide and/or macrolide analog.
40. Use of at least one macrolide and/or macrolide analog with neuro-stimulatory and/or neuro-protective activity, for the preparation of a medicament for the amelioration of a post-ocular surgery patient and the increase of viability and/or activity of retinal sensory or ganglion cells.
41. The method of clause 1 wherein the neuro-protective and/or neuro-stimulatory factor is cyclosporin A.
42. The method of clause 1 wherein the neuro-protective and/or neuro-stimulatory factor is tacrolimus.
43. The method of clause 1 wherein the neuro-protective and/or neuro-stimulatory factor is sirolimus.
44.The method of clause 1 wherein the neuro-protective and/or neuro-stimulatory factor is everolimus.
45.The method of clause 1 wherein the neuro-protective and/or neuro-stimulatory factor is pimecrolimus.
46. The method of clause 1 wherein the neuro-protective and/or neuro-stimulatory factor is zotarolimus.
47. The method of clause 1 wherein the neuro-protective and/or neuro-stimulatory factor is at least one of erythromycin, azithromycin, clarithromycin, lincomycin, dirithromycin, josamycin, spiramycin, diacetyl-midecamycin, troleandomycin, tylosin, roxithromycin, ABT-773, telithromycin, zotarolimus, macrolides derived from leucomycins, lincosamides, or derivatives thereof.
48. Use of at least one of erythromycin, azithromycin, clarithromycin, lincomycin, dirithromycin, josamycin, spiramycin, diacetyl-midecamycin, troleandomycin, tylosin, roxithromycin, ABT-773, telithromycin, zotarolimus, macrolides derived from leucomycins, lincosamides, or derivatives thereof for preparation of a medicament for enhancing ocular neuroprotection and/or neurostimulation in or to the eye of a patient.
49. The method of clause 1 wherein a macrolide and/or macrolide analog is administered in conjunction with acetazolamide.
50. An ocular method comprising locally administering to an eye of an individual a biocompatible formulation of at least one neurostimulatory and/or neuroprotective macrolide and/or macrolide analog, optionally further including at least one neurotrophin or neuropoietic factor, under conditions to result in enhanced viability and/or activity of retinal sensory or ganglion cells over viability and/or activity of the cells in the absence of the macrolide and/or macrolide analog.
51. Use of at least one neuro-stimulatory factor and a neurotrophin or neuropoietic factor, for the preparation of a medicament for the amelioration of a patient for the increased viability and/or activity of retinal sensory or ganglion cells.
52. The method of clause 50 administered to a patient having or at risk for developing glaucoma.
53. The method of clause 50 administered to a patient having or at risk for developing retinitis pigmentosa.
54. A composition comprising at least one neuro-stimulatory and/or neuro-protective factor in a pharmaceutically effective concentration and formulation for non-systemic localized ocular administration and effect wherein the composition contains macrolides and/or macrolid analogs or may further be modified to contain one or more macrolides and/or macrolide analogs, if not already present.
55. A composition, comprising: at least one neuro-protective and/or neuro-stimulatory factor in a pharmaceutically effective concentration and formulation for localized non-systemic ocular administration and effect.
56. A composition comprising at least one of a neurotrophin, neuropoietic factor, or macrolide analog with neuro-stimulatory activity in a pharmaceutically effective concentration and formulation for non-systemic localized ocular administration.
57.The composition of clause 56 further comprising a macrolide.
58. The composition of clause 56 formulated with excipients for at least one of topical ocular administration, subconjunctival administration, or intraocular injection.
59. The composition of clause 56 contained in an intraocular implant, an intraocular lens, or a contact lens.
60. The composition of clause 56 wherein the macrolide is cyclosporin A.
61. The composition of clause 56 wherein the macrolide is tacrolimus.
62.The composition of clause 56 wherein the macrolide is sirolimus.
63.The composition of clause 56 wherein the macrolide is everolimus.
64.The composition of clause 56 wherein the macrolide is pimecrolimus.
65.The composition of clause 56 wherein the macrolide is zotarolimus.
66. The composition of clause 56 wherein the macrolide is at least one of erythromycin, azithromycin, clarithromycin, lincomycin, dirithromycin, josamycin, spiramycin, diacetyl-midecamycin, troleandomycin, tylosin, roxithromycin, ABT-773, telithromycin, zotarolimus, macrolides derived from leucomycins, lincosamides, or derivatives thereof.
67. The composition of clause 56 wherein the neurotrophin is at least one of nerve growth factor-β (NGFβ), brain-derived neurotrophic factor (BDNF), neurotrophin 3 (NT-3), neurotrophin 4 (NT-4), neurotrophin 6, ciliary neurotrophic factor (CNTF), or glial cell derived neurotrophic factor (GDNF), and the neuropoietic factor is at least one of leukemia inhibitory factor (LIF), ciliary neurotrophic factor (CNTF), oncostatin M, growth-promoting activity, or cardiotrophin 1.
68. The composition of clause 56 further comprising at least one of a steroid, non-steroidal anti-inflammatory drug, antibiotic, anti-proliferative agent, anti-cell migration agent, anti-prostaglandin, anti-angiogenic agent, vitamin, mineral, growth factor, or cytokine.
69. An ocular method comprising administering to a patient a composition comprising at least one of a neurotrophin, neuropoietic factor, or macrolide or macrolide analog with neuro-stimulatory and/or neuro-protective activity in a pharmaceutically effective concentration and formulation for non-systemic localized ocular administration for a duration sufficient to enhance the patents corneal sensation.
70. The method of clause 69 wherein administration is by topical ocular administration, subconjunctival administration, or intraocular injection.
71. The method of clause 69 wherein administration is from an ocular implant, a intraocular lens, or a contact lens.
72. The method of clause 69 wherein the composition is administered after corneal surgery.
73. The method of clause 69 wherein the composition is administered after at least one of laser-assisted *in situ* keratomileusis (LASIK), photorefractive keratectomy (PRK), total corneal transplant, or partial corneal transplant.
74. The method of clause 69 wherein the composition comprises cyclosporin A.
75. The method of clause 69 wherein the composition comprises tacrolimus.
76. The method of clause 69 wherein the composition comprises sirolimus.
77.The method of clause 69 wherein the composition comprises everolimus.
78. The method of clause 69 wherein the composition comprises pimecrolimus.
79. The method of clause 69 wherein the composition comprises zotarolimus.
80. The method of clause 69 wherein the macrolide is at least one of erythromycin, azithromycin, clarithromycin, lincomycin, dirithromycin, josamycin, spiramycin, diacetyl-midecamycin, troleandomycin, tylosin, roxithromycin, ABT-773, telithromycin, zotarolimus, macrolides derived from leucomycins, lincosamides, or derivatives thereof.
81. " The method of clause 13 wherein the composition is administered to a diabetic patient.
82. A method for enhancing corneal sensation in a patient following ocular surgery, the method comprising administering to the patient an effective amount of a composition comprising an agent with neuro-stimulatory and/or neuro-protective activity, the agent selected from at least one of a macrolide, a macrolide analog, a neurotrophin, or a neuropoietic factor, the agent in a pharmaceutically acceptable formulation for ocular administration and effective concentration to enhance the patient's post-ocular surgery corneal sensation.
83. A method comprising administering to a patient after LASIK surgery a composition comprising at least one of a macrolide and/or macrolide analog with neuro-stimulatory and/or neuro-protective activity, a neurotrophin, or a neuropoietic factor, in a pharmaceutically effective concentration and formulation for non-systemic localized ocular administration by a method selected from topical administration, subconjunctival administration, intraocular injection, ocular implantation, or contact lens delivery, at a dose and for a duration sufficient to enhance the patient's corneal sensation.
84. An ocular method comprising administering to a patient after ocular surgery a composition comprising at least one macrolide and/or a macrolide analog, with neuro-stimulatory and/or neuro-protective activity, in a pharmaceutically effective concentration and formulation for non-systemic localized ocular administration for a duration sufficient to reduce post surgical ocular scarring.
85. The method of clause 84 wherein administration is selected from at least one of topical, subconjunctival, or intraocular.
86. The method of clause 84 1 wherein the ocular surgery is at least one of glaucoma surgery, retinal detachment repair surgery, or corneal surgery.
87. The method of clause 84 wherein the macrolide is cyclosporin A.
88. The method of clause 84 wherein the macrolide is tacrolimus.
89. The method of clause 84 wherein the macrolide is sirolimus.
90. The method of clause 84 wherein the macrolide is everolimus.
91. The method of clause 84 wherein the macrolide is pimecrolous.
92. The method of clause 84 wherein the macrolide is zotarolimus.
93. The method of clause 84 wherein the macrolide is at least one of erythromycin, azithromycin, clarithromycin, lincomycin, dirithromycin, josamycin, spiramycin, diacetyl-midecamycin, troleandomycin, tylosin, roxithromycin, ABT-773, telithromycin, zotarolimus, macrolides derived from leucomycins, lincosamides, or derivatives thereof

## Claims

1. A macrolide analog with neuro-stimulatory and/or neuro-protective activity for use in partially or completely enhancing restoration of corneal sensation in the eye of a patient following laser assisted *in situ* keratomileusis (LASIK), wherein the administration is non-systemic localised ocular administration.

2. The macrolide analog for use according to claim 1 wherein the administration is topical, intraocular injection, sub-conjunctival administration or intravitreal application.

3. The macrolide analog for use according to claim 1 or 2 wherein the macrolide analog is formulated as a delayed or extended-release formulation.

4. The macrolide analog for use according to claim 1 wherein the macrolide analog is formulated in a lens.

5. The macrolide analog for use according to any one of the preceding claims wherein the macrolide analog is formulated in a liposome, microsphere, microcapsule, biocompatible matrix gel, polymer, nano particle or nano capsule.

6. The macrolide analog for use according to any one of the preceding claims wherein the macrolide analog is present in an amount of less than 1ng/ml to 10mg/ml, preferably from 1ng/ml to 1mg/ml.

7. The macrolide analog for use according to any one of the preceding claims wherein the macrolide analog is an analog of ascomycin that contains hydrogen, methyl or ethyl groups, instead of a methoxy group, at either the 13-, the 15-, or both the 13- and 15- positions.

8. Use of a macrolide analog with neuro-stimulatory and/or neuro-protective activity for the preparation of a medicament for administration under conditions for partially or completely enhancing restoration of corneal sensation in the eye of a patient following laser assisted *in situ* keratomileusis (LASIK), wherein the medicament is formulated for non-systemic localised ocular administration.

9. The use according to claim 8 wherein the medicament is formulated for topical, intraocular injection, sub-conjunctival administration or intravitreal application.

10. The use according to claim 8 or 9 wherein the medicament is a delayed or extended-release formulation.

11. The use according to claim 8 wherein the medicament is formulated in a lens.

12. The use according to any one of claims 8 to 11 wherein the medicament is formulated as a liposome, microsphere, microcapsule, biocompatible matrix gel, polymer, nano particle or nano capsule.

13. The use according to any of claims 8 to 12 wherein the total macrolide concentration of the medicament is from less than 1ng/ml to 10 mg/ml, preferably from 1ng/ml to 1 mg/ml.

14. The use according to any one of the claims 8 to 13 wherein the macrolide analog is an analog of ascomycin that contains hydrogen, methyl or ethyl groups, instead of a methoxy group, at either the 13-, the 15-, or both the 13- and 15- positions.
